# EUROPEAN PATENT APPLICATION

(11) **EP 4 411 744 A1**
(43) Date of publication of application: **07.08.2024**
(21) Application number: 24152726.6
(22) Date of filing: 18.01.2024
(51) Int. Cl.: G16H 20/00, G16H 50/20, A61B 5/00, G06T 7/00, A61C 19/04

(54) **SUPPORT APPARATUS, SUPPORT METHOD, AND SUPPORT PROGRAM**

(30) Priority: 01.02.2023 JP 2023013834
(71) Applicant: J. MORITA MANUFACTURING CORPORATION, Fushimi-ku, Kyoto-shi, Kyoto 6128533 (JP)
(72) Inventor: YOSHIKAWA, Hideki, Kyoto, 612-8533 (JP); NISHIMURA, Yuu, Kyoto, 612-8533 (JP)
(74) Representative: Müller Hoffmann & Partner

(57) **Abstract**

A support apparatus (1) includes an input interface (14) that receives input of image data showing a three-dimensional geometry of a biological tissue and a computing device (11) that derives support information including information on positions of at least the tooth and the gingiva relative to each other, by using the image data inputted from the input interface (14) and an estimation model (50) for support of diagnosis of the state of disease in the biological tissue based on the image data, the estimation model being trained by machine learning to derive the support information.

## Description

### CROSS REFERENCE TO RELATED APPLICATIONS

This application is based on Japanese Patent Application No. 2023-013834 filed with the Japan Patent Office on February 1, 2023.

### BACKGROUND OF THE INVENTION

### Field of the Invention

The present disclosure relates to a support apparatus, a support method, and a support program that support diagnosis of a state of disease in a biological tissue in an oral cavity including at least a tooth and a gingiva.

### Description of the Background Art

A periodontal disease is an infectious inflammatory disease caused by periodontal pathogenic bacteria. The periodontal disease includes diseases caused in the periodontium composed of a gingiva, cementum, a periodontal membrane, and an alveolar bone, necrotizing periodontal diseases, abscesses of the periodontium, combined periodontic-endodontic lesions, gingival recession (for example, lowering of the gingiva), recession of the alveolar bone (for example, lowering of the alveolar bone), and occlusal trauma caused by strong occlusal force or abnormal force. Progress of the periodontal disease causes inflammation of the gingiva, tooth mobility, or recession of the alveolar bone.

A method of measuring a depth of a periodontal pocket has been known as a method of diagnosing a state of disease in a biological tissue in an oral cavity, such as a tooth and a gingiva. For example, Japanese Patent Laying-Open No. 10-174693 discloses examination of a state of a tooth and a gingiva by measurement by an operator such as a dentist, of a depth of a periodontal pocket by insertion of a hand-held probe into the periodontal pocket.

### SUMMARY OF THE INVENTION

In a method of examination for a periodontal disease disclosed in Japanese Patent Laying-Open No. 10-174693, the probe should be inserted in the periodontal pocket, which imposes great burdens on a patient. In addition, a result of examination for the periodontal disease may vary depending on skills of an operator who measures the depth of the periodontal pocket. The probe is inserted in and taken out of the periodontal pocket where pathogenic bacteria are present. Therefore, when the pathogenic bacteria enter a tooth with minor or no symptom via the probe, the tooth may be infected by the periodontal disease. Furthermore, in measurement of the depth of the periodontal pocket, the patient may feel pain or experience bleeding. When the patient experiences bleeding, the pathogenic bacteria may also enter the blood. When the depth of the periodontal pocket is measured at a plurality of locations of each tooth, a time period for measurement may be long.

The present disclosure was made to solve such problems, and an object thereof is to provide a technique for less-invasive diagnosis of a state of disease in a biological tissue in an oral cavity in a short period of time.

According to an example of the present disclosure, a support apparatus that supports diagnosis of a state of disease in a biological tissue in an oral cavity including at least a tooth and a gingiva is provided. The support apparatus includes an input unit that receives input of image data showing a three-dimensional geometry of the biological tissue and a computing unit that derives support information including information on positions of at least the tooth and the gingiva relative to each other, by using the image data inputted from the input unit and an estimation model for support of diagnosis of the state of disease in the biological tissue based on the image data, the estimation model being trained by machine learning to derive the support information.

According to an example of the present disclosure, a support method of supporting, by a computer, diagnosis of a state of disease in a biological tissue in an oral cavity including at least a tooth and a gingiva is provided. The support method includes, as processing to be performed by the computer, obtaining image data showing a three-dimensional geometry of the biological tissue and deriving support information including information on positions of at least the tooth and the gingiva relative to each other, by using the image data obtained in the obtaining of image data and an estimation model for support of diagnosis of the state of disease in the biological tissue based on the image data, the estimation model being trained by machine learning to derive the support information.

According to an example of the present disclosure, a support program for support, by a computer, of diagnosis of a state of disease in a biological tissue in an oral cavity including at least a tooth and a gingiva is provided. The support program causes the computer to perform obtaining image data showing a three-dimensional geometry of the biological tissue and deriving support information including information on positions of at least the tooth and the gingiva relative to each other, by using the image data obtained in the obtaining of image data and an estimation model for support of diagnosis of the state of disease in the biological tissue based on the image data, the estimation model being trained by machine learning to derive the support information.

The foregoing and other objects, features, aspects and advantages of this invention will become more apparent from the following detailed description of this invention when taken in conjunction with the accompanying drawings.

### BRIEF DESCRIPTION OF THE DRAWINGS

Fig. 1 is a diagram showing an application of a support apparatus.
Fig. 2 is a block diagram showing a hardware configuration of the support apparatusof Fig. 1.
Figs. 3 and 4 are each a diagram for illustrating a parameter estimated in the support apparatus of Fig. 1.
Fig. 5 is a diagram for illustrating what is detected in each of CT data and IOS data.
Fig. 6 is a diagram for illustrating exemplary machine learning in a training phase of an estimation model.
Fig. 7 is a diagram for illustrating exemplary estimation of support information in a utilization phase of the estimation model of Fig. 6.
Fig. 8 is a flowchart for illustrating exemplary support processing performed by the support apparatus of Fig. 1.
Fig. 9 is a diagram for illustrating first exemplary representation of support information by the support apparatus of Fig. 1.
Fig. 10 is a diagram for illustrating second exemplary representation of the support information by the support apparatus of Fig. 1.
Fig. 11 is a diagram for illustrating third exemplary representation of the support information by the support apparatus of Fig. 1.
Fig. 12 is a diagram for illustrating fourth exemplary representation of the support information by the support apparatus of Fig. 1.
Fig. 13 is a diagram for illustrating fifth exemplary representation of the support information by the support apparatus of Fig. 1.
Fig. 14 is a diagram for illustrating exemplary machine learning in the training phase of the estimation model according to a first modification.
Fig. 15 is a diagram for illustrating exemplary estimation of the support information in the utilization phase of the estimation model according to the first modification.
Fig. 16 is a diagram for illustrating exemplary machine learning in the training phase of the estimation model according to a second modification.
Fig. 17 is a diagram for illustrating exemplary estimation of the support information in the utilization phase of the estimation model according to the second modification.
Fig. 18 is a diagram for illustrating exemplary machine learning in the training phase of the estimation model according to a third modification.
Fig. 19 is a diagram for illustrating exemplary estimation of the support information in the utilization phase of the estimation model according to the third modification.
Fig. 20 is a diagram for illustrating exemplary machine learning in the training phase of another estimation model.
Fig. 21 is a diagram for illustrating exemplary estimation of the support information in the utilization phase of the estimation model of Fig. 20.
Fig. 22 is a diagram for illustrating exemplary output of the support information by the support apparatus.
Figs. 23 and 24 are each a diagram for illustrating a parameter estimated in another support apparatus.

### DESCRIPTION

The present disclosure will be described in detail with reference to the drawings. The same or corresponding elements in the drawings have the same reference characters allotted and description thereof will not be repeated.

### [Application]

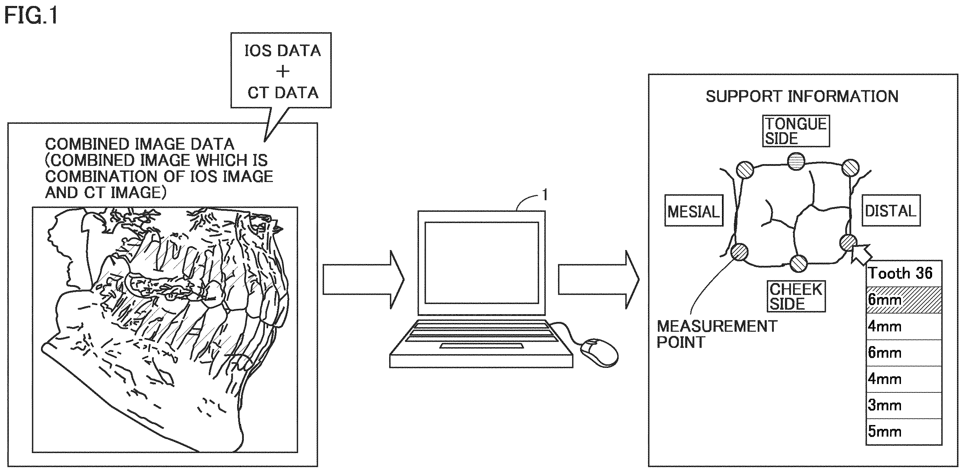

An application of a support apparatus 1 will be described with reference to Fig. 1. Fig. 1 is a diagram showing an application of support apparatus 1

A method of measuring a depth of a periodontal pocket is available as a method most used in examination for a periodontal disease. Specifically, an operator such as a dentist can measure the depth of the periodontal pocket by inserting a mechanical or electrical probe into the periodontal pocket present at at least one measurement point set around a tooth and measuring the depth from a gingival margin to a gingival junction (a bottom of the periodontal pocket) which is a portion where the gingiva is normally attached to the tooth. Any number of measurement points, for example, four or six measurement points, can be set for one tooth. In the example in Fig. 1, six measurement points are set for a single tooth.

Other than the method of measuring the depth of the periodontal pocket, a method of using a radiograph obtained by radiographing the oral cavity is available as the method of examination for the periodontal disease. This method includes measurement of a degree of recession of an alveolar bone by measurement of a distance between a crown top and an alveolar bone top and a distance between the alveolar bone top and a root apex portion based on the radiograph of the oral cavity and calculation of a ratio therebetween (which is also referred to as a "crown-root ratio" below).

As described above, the operator can conduct examination for the periodontal disease based on measurement of the periodontal pocket and the radiograph. These examination methods, however, may not be preferred as the examination for the periodontal disease from a point of view of burdens imposed on a patient, variation in examination accuracy depending on skills of the operator, and a long time period required for examination.

For example, in the method of examination for the periodontal disease based on measurement of the periodontal pocket, the probe should be inserted in the periodontal pocket, which imposes great burdens on a patient. In addition, a result of examination for the periodontal disease may vary depending on skills of the operator who measures the depth of the periodontal pocket, and diagnosis by the operator of a plurality of parameters for diagnosis of the periodontal disease based on his/her experiences and prediction of progress of the periodontal disease require considerable experiences and time. The probe is inserted in and taken out of the periodontal pocket where pathogenic bacteria are present. Therefore, when the pathogenic bacteria enter a tooth with minor or no symptom via the probe, the tooth may be infected by the periodontal disease. Furthermore, in measurement of the depth of the periodontal pocket, the patient may feel pain or experience bleeding. When the patient experiences bleeding, the pathogenic bacteria may also enter the blood. When the depth of the periodontal pocket is measured at a plurality of locations of each tooth, a time period for measurement may be long.

In the method of examination for the periodontal disease with the use of the radiograph, intraoral radiography or panorama radiography is performed. In this case, however, only a state of a tooth when viewed in a direction of incidence of X rays can be observed, and hence an image superimposed in the direction of incidence of X rays is shown. Therefore, it is difficult to accurately measure the crown-root ratio.

For the reasons as described above, in development of future therapeutic strategies, it is difficult to quantitatively accumulate data on results of examination for the periodontal disease and also to estimate a degree of progress of the periodontal disease over time. Furthermore, in explanation of a state of the periodontal disease to a patient, such a technique as obtaining informed consent and understanding by the patient in a short period of time is also required.

Support apparatus 1 is configured to estimate (derive), with the use of artificial intelligence (AI) technology, support information for support of diagnosis of a state of disease in a biological tissue in an oral cavity including at least a tooth and a gingiva based on image data showing a three-dimensional geometry of the biological tissue. The biological tissue is an object in the oral cavity including at least the tooth and the gingiva and does not include an artificial article such as an implant.

Specifically, a user of support apparatus 1 obtains three-dimensional data (optical scanner data) including position information of each point in a point group (a plurality of points) indicating a surface of the biological tissue including the tooth and the gingiva in the oral cavity by scanning the inside of the oral cavity of a patient with the use of a not-shown three-dimensional scanner (optical scanner). The three-dimensional data includes as the position information, a coordinate (X, Y, Z) of each point indicating the surface of the biological tissue in a lateral direction (an X-axis direction), a longitudinal direction (a Y-axis direction), and a height direction (a Z-axis direction) that are predetermined. The three-dimensional data may include color information indicating an actual color of a portion (a surface portion of the biological tissue) corresponding to each point in the point group (the plurality of points) indicating the surface of the biological tissue including the tooth and the gingiva in the oral cavity.

The "user" includes an operator (a doctor or the like) or an assistant (a dental nurse, a dental technician, a nurse, or the like) in various fields such as dentistry, dental surgery, orthopedics, plastic surgery, and cosmetic surgery. The "patient" includes a patient of dentistry, dental surgery, orthopedics, plastic surgery, and cosmetic surgery. The three-dimensional scanner is what is called an intraoral scanner (IOS) capable of optically picking up an image of the inside of the oral cavity of a patient by a confocal method or triangulation, and it is capable of obtaining position information of each point in the point group that defines the surface (for example, the tooth and the gingiva in the oral cavity) of the biological tissue set in a certain coordinate space that is to be scanned. The user can generate a rendered image (an appearance image) showing a three-dimensional geometry of the biological tissue based on three-dimensional data obtained by the three-dimensional scanner. The "rendered image" is an image generated by processing or edition of certain data. For example, the user can generate a rendered image showing a two-dimensional biological tissue (a part of the biological tissue that can be shown based on IOS data) viewed from a prescribed point of view by processing or edition of three-dimensional data of the biological tissue obtained by the three-dimensional scanner, and can generate, by varying the prescribed point of view in multiple directions, a plurality of rendered images showing the two-dimensional biological tissue (a part of the biological tissue that can be shown based on IOS data) viewed in multiple directions.

The user obtains three-dimensional volume (voxel) data of a hard tissue portion (a bone, a tooth, or the like) including a soft tissue portion (the skin, the gingiva, or the like) around a maxilla and a mandible of a patient by scanning the maxilla and the mandible of the patient with a not-shown computed tomography (CT) scanner. The soft tissue portion is detected with X rays less clearly than the hard tissue portion, and accuracy in obtainment of data thereon is low. Therefore, when the soft tissue portion is shown in an image, the soft tissue portion can visually be recognized vaguely or it may partially be visually unrecognizable. The CT scanner is X-ray equipment that takes a CT of the maxilla and the mandible of the patient by rotation around the face of the patient, of a transmitter and a receiver of X rays which are a kind of radioactive rays. The user can generate the rendered image (a CT image or an appearance image) showing the three-dimensional geometry of the biological tissue based on volume data obtained by the CT scanner, of the biological tissue which is a scan target. For example, the user can generate a rendered image showing the two-dimensional biological tissue (a part of the biological tissue that can be shown based on the CT data) viewed from a prescribed point of view by processing or edition of volume data of the biological tissue obtained by the CT scanner, and further generate, by varying the prescribed point of view in multiple directions, a plurality of rendered images showing the two-dimensional biological tissue (a part of the biological tissue that can be shown based on the CT data) viewed from multiple directions.

The three-dimensional data including position information of each point in a point group indicating the surface of the biological tissue, the position information being obtained by the three-dimensional scanner, is also referred to as "IOS data," and the rendered image generated based on the IOS data is also referred to as an "IOS image" below. The three-dimensional volume data obtained by the CT scanner is also referred to as "CT data" and the rendered image generated based on the CT data is also referred to as a "CT image." The IOS image can show in a very detailed manner, a surface geometry of the biological tissue to be scanned, whereas it cannot show an internal construction (the alveolar bone, the root apex portion, or the like) that does not appear at the surface of the biological tissue. The CT image can show the hard tissue portion (the bone, the tooth, or the like) of a scan target relatively in detail, whereas it cannot show the soft tissue portion (the skin, the gingiva, or the like) in a manner as detailed as the hard tissue portion.

The user can generate combined image data by combining the IOS data and the CT data obtained for the same patient. The IOS data and the CT data are different from each other in data format. Therefore, the user generates the combined image data which is combination of the IOS data and the CT data, for example, by converting the data format of the IOS data into the data format of the CT data and subjecting the three-dimensional geometry of the surface of the biological tissue to pattern matching with the use of converted data of both of them. Alternatively, the user may generate the combined image data by converting the data format of the CT data into the data format of the IOS data and subjecting the three-dimensional geometry of the surface of the biological tissue to pattern matching with the use of converted data of both of them. Alternatively, the user may generate the combined image data by converting the data formats of the CT data and the IOS data into a common data format and subjecting the three-dimensional geometry of the surface of the biological tissue to pattern matching with the use of converted data of both of them. The user can generate a rendered image (for example, a combined image shown in Fig. 1) showing a two-dimensional biological tissue (a part of the biological tissue that can be shown based on both of the IOS data and the CT data) viewed from a prescribed point of view by processing or edition of the combined image data. As shown in Fig. 1, the combined image can three-dimensionally show the surface geometry of the biological tissue based on the IOS data and a tomographic structure or an appearance in the hard tissue portion (the bone, the tooth, or the like) based on the CT data. In generation of the combined image data, the user may adjust a luminance, a contrast, a transmittance, and the like as necessary in each of the IOS data and the CT data. Furthermore, the user may generate the combined image data by advance segmentation of each of teeth, the jaw bone, and the alveolar bone in each of the IOS data and the CT data.

Support apparatus 1 may obtain the IOS data from the three-dimensional scanner, obtain the CT data from the CT scanner, and generate the combined image data based on the obtained IOS data and CT data in accordance with input from the user. Alternatively, support apparatus 1 may obtain from another apparatus, the combined image data generated by the user with another apparatus, without obtaining the IOS data and the CT data.

The combined image that can be generated based on the combined image data shows based on the CT data, the three-dimensional geometry of the hard tissue portion such as the alveolar bone and the root apex portion and shows based on the IOS data, the three-dimensional geometry of the soft tissue portion such as the gingiva that cannot be shown based on the CT data. The combined image can thus show in detail also the soft tissue portion such as the gingiva that cannot be shown only based on the CT data, owing to supplement by the IOS data, together with the hard tissue portion such as the alveolar bone and the root apex portion.

Though detailed description will be given later, support apparatus 1 sets a prescribed direction of measurement and a prescribed measurement point for measurement of the depth of the periodontal pocket in the combined image data of the biological tissue including at least the tooth and the gingiva. Support apparatus 1 uses an estimation model 50 which will be described later to derive support information for support of diagnosis of a state of disease in the biological tissue, the support information including information on positions of at least the tooth and the gingiva relative to each other.

As shown in Fig. 1, for example, support apparatus 1 shows as the support information, the depth of the periodontal pocket at the set measurement point for each tooth shown based on the combined image data. Since support apparatus 1 can thus derive the support information for support of diagnosis of the state of disease in the biological tissue based on the combined image data showing the three-dimensional geometry of the biological tissue including at least the tooth and the gingiva, the user can less invasively make diagnosis of the state of disease in the biological tissue in the oral cavity in a short period of time.

### [Hardware Configuration of Support Apparatus]

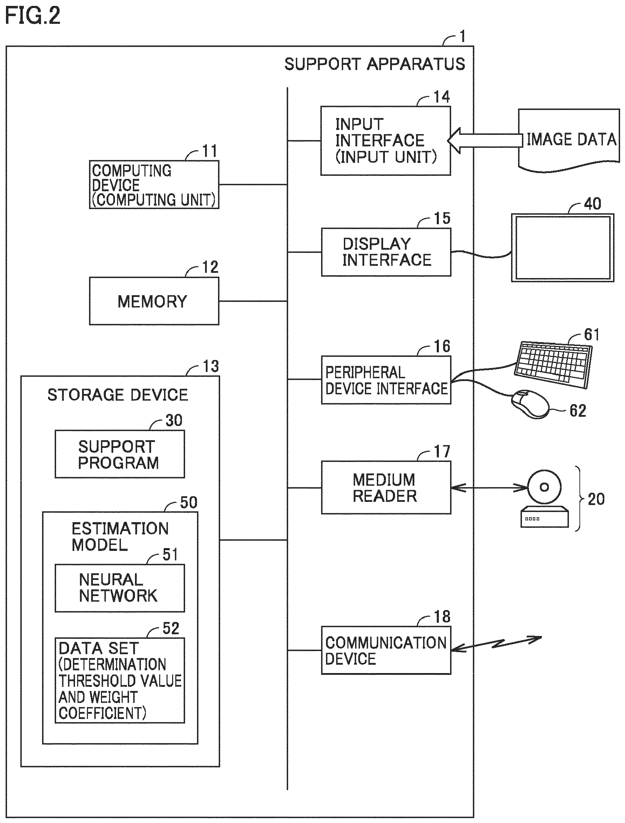

A hardware configuration of the support apparatus 1 of Fig. 1 will be described with reference to Fig. 2. Fig. 2 is a block diagram showing a hardware configuration of the support apparatus 1. Support apparatus 1 may be implemented, for example, by a general-purpose computer or a computer dedicated for a system for estimation of the support information.

As shown in Fig. 2, support apparatus 1 includes, as its main hardware elements, a computing device 11, a memory 12, a storage device 13, an input interface 14, a display interface 15, a peripheral device interface 16, a medium reader 17, and a communication device 18.

Computing device 11 is a computing entity (computer) that performs various types of processing by executing various programs, and represents an exemplary "computing unit." Computing device 11 is implemented by a processor such as a central processing unit (CPU) or a micro-processing unit (MPU). Though the processor which represents an exemplary computing device 11 performs functions to perform various types of processing by executing a program, some or all of these functions may be performed by dedicated hardware circuitry such as an application specific integrated circuit (ASIC) or a field-programmable gate array (FPGA). The "processor" is not limited to a processor in a narrow sense that performs processing in accordance with a stored program architecture like the CPU or the MPU, but may encompass hard-wired circuitry such as the ASIC or the FPGA. Therefore, the "processor" representing exemplary computing device 11 can also be read as processing circuitry, processing by which is defined in advance by a computer readable code and/or hard-wired circuitry. Computing device 11 may be implemented by a single chip or a plurality of chips. Furthermore, the processor and relating processing circuitry may be implemented by a plurality of computers connected to one another through wires or wirelessly over a local area network or a wireless network. The processor and the relating processing circuitry may be implemented by a cloud computer that performs remote computation based on input data and outputs a result of computation to another device located at a remote position.

Memory 12 includes a volatile storage area (for example, a working area) where a program code or a work memory is temporarily stored in execution of various programs by computing device 11. Examples of a storage unit include a volatile memory such as a dynamic random access memory (DRAM) and a static random access memory (SRAM) or a non-volatile memory such as a read only memory (ROM) and a flash memory.

Various programs to be executed by computing device 11 or various types of data are stored in storage device 13. Storage device 13 may be implemented by one or more non-transitory computer readable media or one or more computer readable storage media. Examples of storage device 13 include a hard disk drive (HDD) and a solid state drive (SSD).

A support program 30 and estimation model 50 are stored in storage device 13. In support program 30, contents of support processing for computing device 11 to estimate support information with the use of estimation model 50 based on image data (for example, combined image data) showing the three-dimensional geometry of the biological tissue are described.

Estimation model 50 includes a neural network 51 and a data set 52 to be used by neural network 51. Estimation model 50 is trained to estimate the support information based on the image data (for example, the combined image data) showing the three-dimensional geometry of the biological tissue in the oral cavity, by machine learning with training data including the image data and the support information associated with the image data.

Any algorithm may be applied to neural network 51, so long as the algorithm is applicable to neural network 51 such as an auto-encoder, a convolutional neural network (CNN), a recurrent neural network (RNN), or a generative adversarial network (GAN). Estimation model 50 may be provided with another known algorithm such as Bayes estimation or support vector machine (SVM), without being limited to neural network 51.

Data set 52 includes a weight coefficient used in computation by neural network 51 and a determination threshold value used for determination at the time of computation.

Input interface 14 is an exemplary "input unit." Input interface 14 obtains the combined image data of the biological tissue including at least the tooth and the gingiva. The combined image data provided from input interface 14 is stored in memory 12 or storage device 13 and used when computing device 11 estimates the support information. Input interface 14 may obtain the IOS data and the CT data yet to be combined. For example, input interface 14 may communicatively be connected to a not-shown three-dimensional scanner and may obtain IOS data from the three-dimensional scanner. Alternatively, input interface 14 may communicatively be connected to a not-shown CT scanner and may obtain CT data from the CT scanner. In this case, computing device 11 generates the combined image data of the biological tissue including the tooth and the gingiva by combining the IOS data and the CT data provided from input interface 14 and estimates the support information based on the generated combined image data.

Display interface 15 is an interface for connection of display 40. Display interface 15 implements input and output of data between support apparatus 1 and display 40.

Peripheral device interface 16 is an interface for connection of a peripheral device such as a keyboard 61 and a mouse 62. Peripheral device interface 16 implements input and output of data between support apparatus 1 and the peripheral device.

Medium reader 17 reads various types of data stored in a removable disc 20 which is a storage medium or writes various types of data into removable disc 20. For example, medium reader 17 may obtain support program 30 from removable disc 20 or write the support information estimated by computing device 11 into removable disc 20. Removable disc 20 may be implemented by one or more non-transitory computer readable media or one or more computer readable storage media. In an example where computing device 11 obtains image data (for example, combined image data) from removable disc 20 through medium reader 17, medium reader 17 can be an exemplary "input unit."

Communication device 18 transmits and receives data to and from an external apparatus through wired or wireless communication. For example, communication device 18 may transmit the support information estimated by computing device 11 to a not-shown external apparatus. In an example where computing device 11 obtains image data (for example, combined image data) from an external apparatus through communication device 18, communication device 18 can be an exemplary "input unit."

### [Parameter Estimated in Support Apparatus]

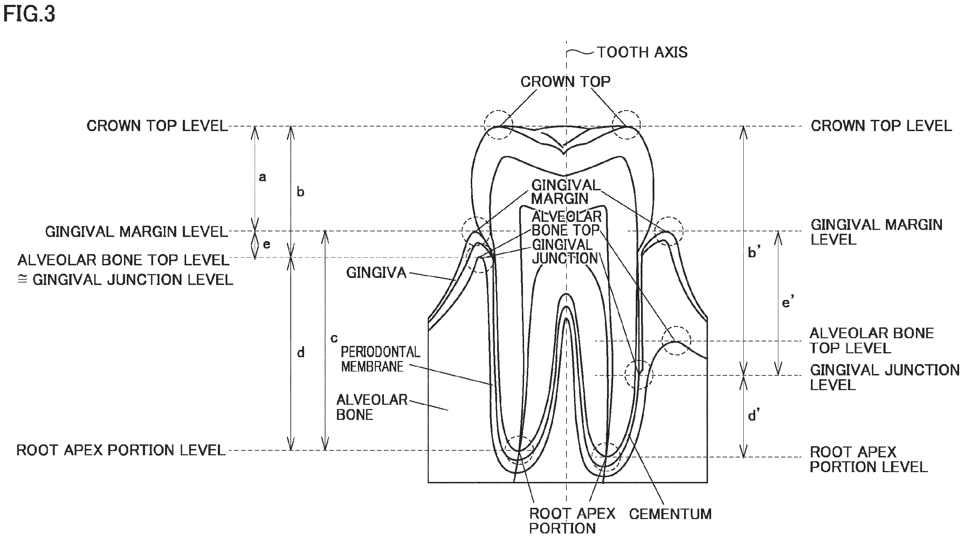

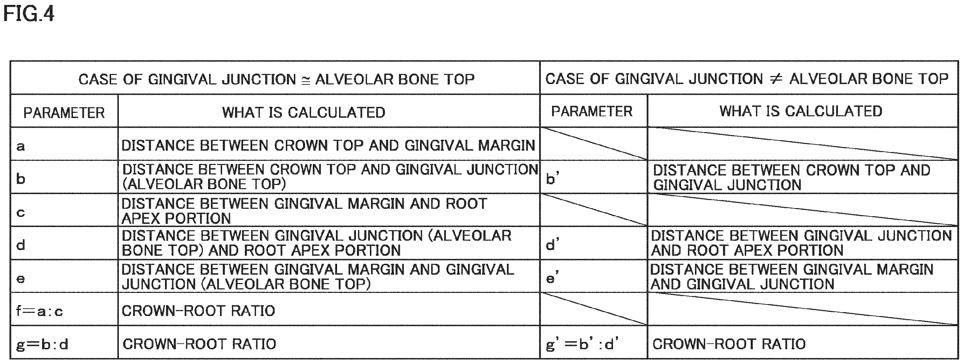

Support apparatus 1 configured as described above is configured to estimate as the support information, various parameters indicating the depth of the periodontal pocket and the like based on the combined image data of the biological tissue including the tooth and the gingiva. The parameters estimated in support apparatus 1 will be described with reference to Figs. 3 and 4. Figs. 3 and 4 are each a diagram for illustrating the parameters estimated in support apparatus 1. Fig. 3 shows a vertical cross-section of the biological tissue including the tooth and the gingiva.

As shown in Fig. 3, support apparatus 1 (computing device 11) sets a prescribed direction of measurement in accordance with a prescribed reference for each tooth in the combined image data of the biological tissue including the tooth and the gingiva. For example, the user designates as the prescribed reference for each tooth, a tooth axis indicating an inclination of the tooth in support apparatus 1, with the use of keyboard 61, mouse 62, and the like while the user looks at the combined image. Support apparatus 1 sets the direction of measurement along a direction of the tooth axis designated by the user. The user may designate as the prescribed reference, a direction other than the direction of the tooth axis in support apparatus 1. In this case, support apparatus 1 sets the direction of measurement along the direction other than the direction of the tooth axis designated by the user. Support apparatus 1 may set the direction of measurement (for example, the tooth axis) with a predetermined mathematical technique based on the geometry of the biological tissue including the tooth and the gingiva shown based on the combined image data.

When support apparatus 1 sets the direction of measurement, it sets at least one prescribed measurement point around each tooth. For example, the user designates at least one measurement point of each tooth in support apparatus 1 with the use of keyboard 61, mouse 62, and the like while the user looks at the combined image. The measurement point is a point located around the tooth when a coronal portion included in the tooth is viewed from the top. Support apparatus 1 sets at least one measurement point designated by the user. Support apparatus 1 may set at least one measurement point with a predetermined mathematical technique based on the geometry of the biological tissue including the tooth and the gingiva shown based on the combined image data.

Support apparatus 1 estimates position information (X, Y, Z) corresponding to each of a plurality of levels indicating a position of the biological tissue present along the direction of measurement at each measurement point of each tooth, with the use of estimation model 50. The plurality of levels described above include a crown top level, a gingival margin level, an alveolar bone top level, a gingival junction level, and a root apex portion level. The crown top level refers to a level corresponding to a position (for example, a height) of a top of the coronal portion in the direction of measurement. The gingival margin level refers to a level corresponding to a position (for example, a height) of a margin of the gingiva in the direction of measurement. The alveolar bone top level refers to a level corresponding to a position (for example, a height) of a top of the alveolar bone in the direction of measurement. The gingival junction level refers to a level corresponding to a position (for example, a height) of a portion where the gingiva is normally attached to the tooth and a junction (the bottom of the periodontal pocket) between the tooth and the gingiva. The root apex portion level refers to a level corresponding to a position (for example, a height) of the root apex portion in the direction of measurement. When there is no periodontal pocket or the depth of the periodontal pocket is extremely small, the gingival junction level and the alveolar bone top level are flush or substantially flush with each other, and a difference between the gingival junction level and the alveolar bone top level is smaller than a prescribed threshold value.

When support apparatus 1 estimates the position information (X, Y, Z) corresponding to each of the plurality of levels present along the direction of measurement, it calculates various parameters based on the estimated position information of each level. At this time, support apparatus 1 calculates different parameters in accordance with whether or not the gingival junction level and the alveolar bone top level are flush or substantially flush with each other.

Specifically, as shown in Figs. 3 and 4, when the gingival junction level and the alveolar bone top level are flush or substantially flush with each other, support apparatus 1 calculates each of parameters a to g. Parameter a is a value indicating a distance along the direction of measurement between the crown top level and the gingival margin level. Parameter b is a value indicating a distance along the direction of measurement between the crown top level and the gingival junction level (alveolar bone top level). Parameter c is a value indicating a distance along the direction of measurement between the gingival margin level and the root apex portion level. Parameter d is a value indicating a distance along the direction of measurement between the gingival junction level (alveolar bone top level) and the root apex portion level. Parameter e is a value indicating a distance along the direction of measurement between the gingival margin level and the gingival junction level (alveolar bone top level). Parameter f is a ratio (a:c) between parameter a and parameter c. Parameter g is a ratio (b:d) between parameter b and parameter d. Parameter f and parameter g are each also referred to as a crown-root ratio.

When the gingival junction level and the alveolar bone top level are not flush or substantially flush with each other, on the other hand, support apparatus 1 calculates each of parameters b', d', e', and g'. Parameter b' is a value indicating a distance along the direction of measurement between the crown top level and the gingival junction level. Parameter d' is a value indicating a distance along the direction of measurement between the gingival junction level and the root apex portion level. Parameter e' is a value indicating a distance along the direction of measurement between the gingival margin level and the gingival junction level. Parameter g' is a ratio (b':d') between parameter b' and parameter d'. Parameter g' is also referred to as the crown-root ratio. When the gingival junction level and the alveolar bone top level are not flush or substantially flush with each other, support apparatus 1 thus skips calculation of the value indicating the distance along the direction of measurement between the crown top level and the gingival margin level corresponding to parameter a and the value indicating the distance along the direction of measurement between the gingival margin level and the root apex portion level corresponding to parameter c. In other words, when the gingival junction level is distant from the alveolar bone top level due to the state of disease, support apparatus 1 may substitute the gingival junction level for the alveolar bone top level or may use the gingival junction level and the alveolar bone top level together.

Though the combined image data is data generated by combination of the CT data and the IOS data as described above, a portion that can be detected from each of the CT data and the IOS data is predetermined.

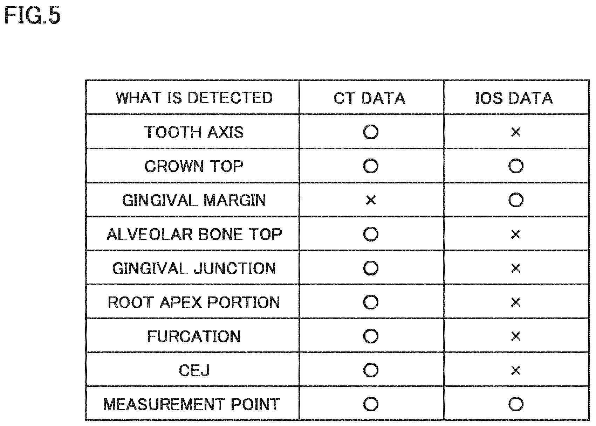

Fig. 5 is a diagram for illustrating what is detected in each of the CT data and the IOS data. As shown by circle in Fig. 5, support apparatus 1 is capable of detecting the tooth axis, the crown top, the alveolar bone top, the gingival junction, the root apex portion, the furcation, a cement enamel junction (CEJ), and the measurement point based on the CT data, whereas as shown by cross in Fig. 5, it is incapable of detecting the gingival margin. The CEJ refers to a junction between cementum located between a dental root and a periodontal membrane and enamel that covers the tooth exposed outside the gingiva, and it is also referred to as a cement enamel junction. Specifically, since the CT data can show the tomographic structure or the appearance in the hard tissue portion (the bone, the tooth, or the like), a portion that can be estimated based on the hard tissue portion can be detected. On the other hand, since the CT data cannot show in detail, the soft tissue portion (the skin, the gingiva, or the like), the gingival margin cannot often sufficiently be detected.

As shown by circle in Fig. 5, support apparatus 1 is capable of detecting the crown top, the gingival margin, and the measurement point based on the IOS data, whereas as shown by cross in Fig. 5, it is incapable of detecting the tooth axis, the alveolar bone top, the gingival junction, the root apex portion, and the CEJ. In other words, since the IOS data can show a surface geometry of the biological tissue regardless of the hard tissue portion and the soft tissue portion, a portion that appears at the surface of the biological tissue can be detected. Since the IOS data, on the other hand, cannot show a portion that does not appear at the surface of the biological tissue, the internal construction of the biological tissue such as the alveolar bone top and the root apex portion cannot be detected.

A portion that can highly accurately be detected based on the CT data and the IOS data is thus predetermined. Support apparatus 1, however, can calculate various parameters a to g, b', d', e', and g' as shown in Fig. 4 with the use of the combined image data which is combination of the CT data and the IOS data. Support apparatus 1 can calculate the various parameters described above for each measurement point for each tooth shown based on the combined image data.

The user can diagnose the state of disease of the periodontal disease for each measurement point of each tooth based on the various parameters estimated by support apparatus 1. For example, an example in which the gingival junction level and the alveolar bone top level are flush or substantially flush with each other implies such a state that the gingiva is not recessed and one has most likely been not affected by the periodontal disease. In this case, the user can check the depth of the periodontal pocket based on parameter e (the distance between the gingival margin level and the gingival junction level (alveolar bone top level)). In addition, the user can check a degree of recession of the gingiva also based on parameter f (a ratio between the distance between the crown top level and the gingival margin level and the distance between the gingival margin level and the root apex portion level) or parameter g (a ratio between the distance between the crown top level and the gingival junction level (alveolar bone top level) and the distance between the gingival junction level (alveolar bone top level) and the root apex portion level).

An example in which the gingival junction level and the alveolar bone top level are not flush or substantially flush with each other implies such a state that the gingiva is recessed and one has most likely been affected by the periodontal disease. In this case, the user can check the depth of the periodontal pocket based on parameter e' (the distance between the gingival margin level and the gingival junction level). In addition, the user can check the degree of recession of the gingiva also based on parameter g' (a ratio between the distance between the crown top level and the gingival junction level and the distance between the gingival junction level and the root apex portion level).

The user can thus low invasively make diagnosis of the state of disease involved with the periodontal disease in the biological tissue including the tooth and the gingiva in a short period of time, by knowing various parameters estimated by support apparatus 1.

### [Training of Estimation Model]

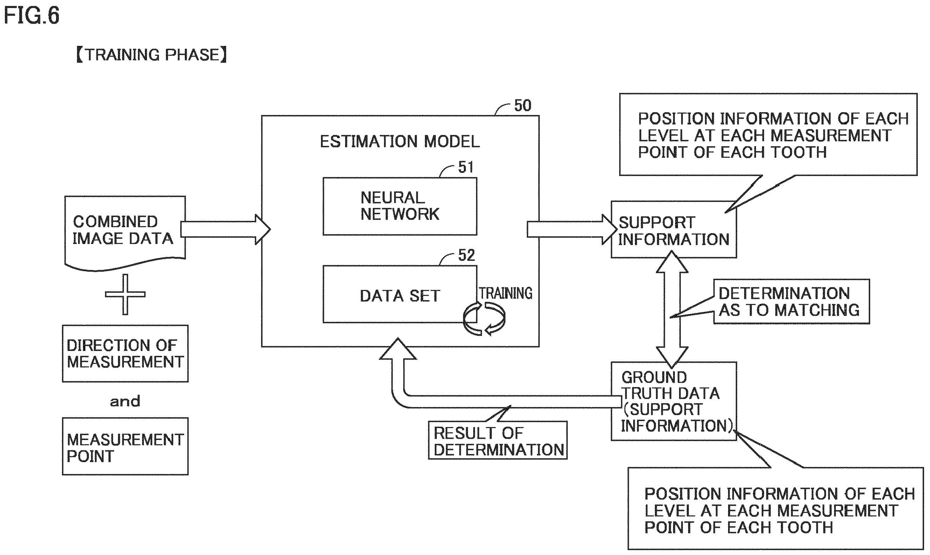

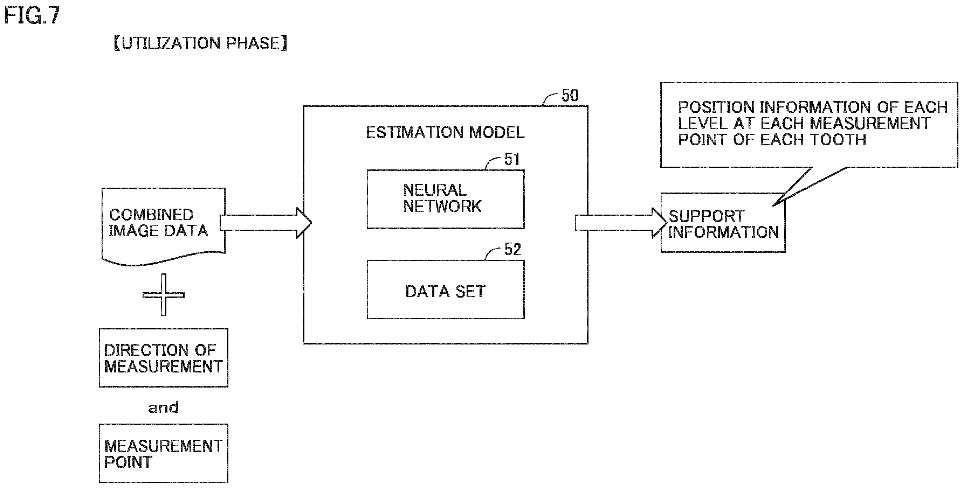

Training of estimation model 50 by machine learning will be described with reference to Figs. 6 and 7. Fig. 6 is a diagram for illustrating exemplary machine learning in a training phase of estimation model 50.

As shown in Fig. 6, the direction of measurement and the measurement point in addition to the combined image data of the biological tissue including the tooth and the gingiva are included in training data. For example, in training of estimation model 50, machine learning is performed with the use of the training data including the combined image data, the direction of measurement, and the measurement point and the support information as ground truth data associated with the combined image data, the direction of measurement, and the measurement point. Position information of each level (the crown top level, the gingival margin level, the alveolar bone top level, the gingival junction level, and the root apex portion level) along the direction of measurement at each measurement point of each tooth is adopted as the support information.

When estimation model 50 receives input of the combined image data, the direction of measurement, and the measurement point, it estimates with neural network 51, the position information (support information) of each level along the direction of measurement at each measurement point of each tooth based on the combined image data, the direction of measurement, and the measurement point. Estimation model 50 determines whether or not the estimated position information (support information) of each level matches with the position information (support information) of each level which is the ground truth data associated with the combined image data, the direction of measurement, and the measurement point. When they match with each other, estimation model 50 does not update data set 52, whereas when they do not match with each other, estimation model 50 updates data set 52 to optimize data set 52.

Estimation model 50 is thus trained to highly accurately estimate the position information of each level (the crown top level, the gingival margin level, the alveolar bone top level, the gingival junction level, and the root apex portion level) along the direction of measurement at each measurement point of each tooth based on the input data, with the use of the training data including the combined image data, the direction of measurement, the measurement point which are input data and the position information (support information) of each level which is the ground truth data to optimize data set 52.

Fig. 7 is a diagram for illustrating exemplary estimation of the support information in a utilization phase of estimation model 50. As shown in Fig. 7, by being trained by machine learning, estimation model 50 can highly accurately estimate as the support information, the position information of each level (the crown top level, the gingival margin level, the alveolar bone top level, the gingival junction level, and the root apex portion level) along the direction of measurement at each measurement point of each tooth, based on the combined image data, the direction of measurement, and the measurement point when it receives input of the combined image data, the direction of measurement, and the measurement point.

### [Data Generation Processing]

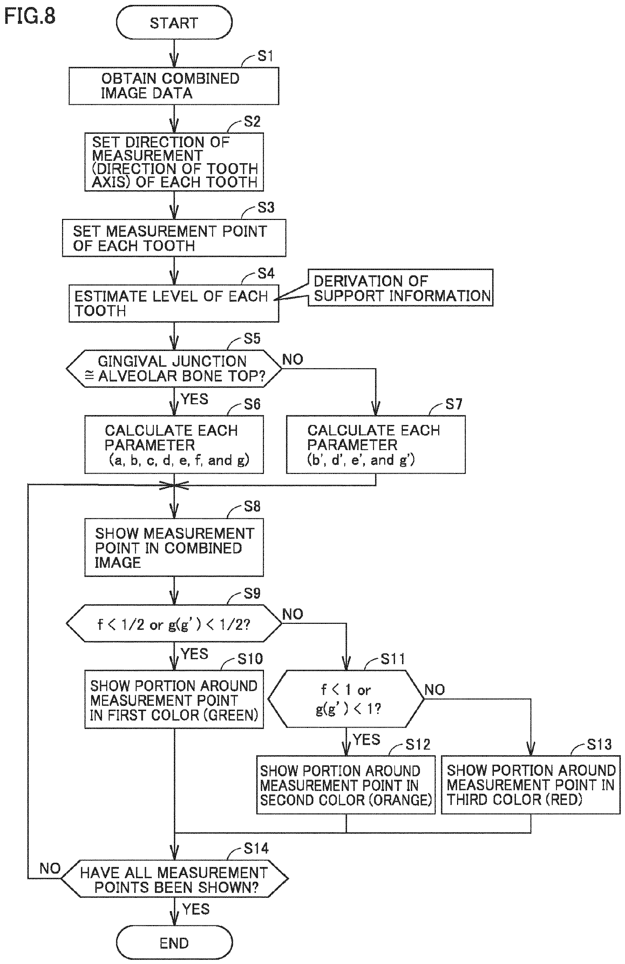

Support processing performed by support apparatus 1 will be described with reference to Fig. 8. Fig. 8 is a flowchart for illustrating exemplary support processing performed by support apparatus 1. Each STEP (which is denoted as "S" below) shown in Fig. 8 is performed by execution of support program 30 by computing device 11 of support apparatus 1.

As shown in Fig. 8, support apparatus 1 obtains the combined image data of the biological tissue including the tooth and the gingiva (S 1). Support apparatus 1 sets the prescribed direction of measurement for each tooth based on the combined image data (S2). For example, support apparatus 1 sets the tooth axis for each tooth as the direction of measurement. At this time, support apparatus 1 sets the measurement direction in accordance with designation by the user. Support apparatus 1 may set the direction of measurement with a predetermined mathematical technique rather than manual setting by the user. When support apparatus 1 automatically sets the direction of measurement rather than manual setting by the user, the user may manually adjust the direction of measurement as necessary with the use of keyboard 61, mouse 62, and the like.

Support apparatus 1 sets the prescribed measurement point for each tooth based on the combined image data (S3). For example, support apparatus 1 sets as the measurement point, a position around the tooth, the depth of the periodontal pocket of which is to be measured generally with the use of a probe, in accordance with designation by the user. Support apparatus 1 may set the measurement point with a predetermined mathematical technique rather than manual setting by the user. When support apparatus 1 automatically sets the measurement point rather than manual setting by the user, the user may manually adjust the measurement point as necessary with the use of keyboard 61, mouse 62, and the like.

Support apparatus 1 estimates with estimation model 50, the position information (X, Y, Z) corresponding to each of the plurality of levels present along the direction of measurement at each measurement point of each tooth (S4). For example, as shown in Fig. 3, support apparatus 1 estimates with estimation model 50, the crown top level, the gingival margin level, the alveolar bone top level, the gingival junction level, and the root apex portion level.

Support apparatus 1 determines whether or not the gingival junction level and the alveolar bone top level are flush or substantially flush with each other based on each estimated level (S5). Specifically, support apparatus 1 determines whether or not the gingival junction level and the alveolar bone top level are flush or substantially flush with each other without recession of the gingiva or whether the gingival junction level and the alveolar bone top level are not flush or substantially flush with each other due to recession of the gingiva. For example, support apparatus 1 determines whether or not a difference between the gingival junction level and the alveolar bone top level is smaller than a prescribed threshold value.

When support apparatus 1 determines that the difference between the gingival junction level and the alveolar bone top level is smaller than the prescribed threshold value and that the gingival junction level and the alveolar bone top level are flush or substantially flush with each other (YES in S5), it calculates each of parameters a to g shown in Fig. 4 (S6). When support apparatus 1 determines that the difference between the gingival junction level and the alveolar bone top level is not smaller than the prescribed threshold value and that the gingival junction level and the alveolar bone top level are not flush or substantially flush with each other (NO in S5), it calculates each of parameters b', d', e', and g' shown in Fig. 4 (S7).

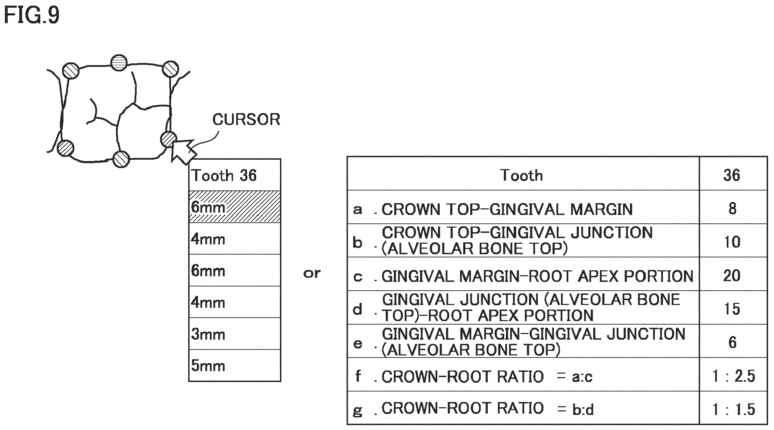

Support apparatus 1 shows on display 40, an image of the biological tissue including the tooth and the gingiva and shows the image with the measurement point being added thereto (S8). For example, as shown in Fig. 1 and Fig. 9 which will be described later, support apparatus 1 shows the image, with six measurement points being added around the tooth in the image.

Furthermore, support apparatus 1 determines the degree of progress of the state of disease of the periodontal disease based on the crown-root ratio (parameter f, g, or g') calculated in S6 and shows a result of determination on display 40.

Specifically, support apparatus 1 determines, for a single measurement point, whether or not the crown-root ratio (parameter f, g, or g') calculated in S6 is smaller than 1/2 (S9). Specifically, when the gingival junction level and the alveolar bone top level are flush or substantially flush with each other, support apparatus 1 determines whether or not parameter f (= a/c) is smaller than 1/2, that is, whether or not a distance (c) between the gingival margin level and the root apex portion level is longer than twice as long as a distance (a) between the crown top level and the gingival margin level. Alternatively, support apparatus 1 determines whether or not parameter g (= b/d) is smaller than 1/2, that is, whether or not a distance (d) between the gingival junction level (alveolar bone top level) and the root apex portion level is longer than twice as long as a distance (b) between the crown top level and the gingival junction level (alveolar bone top level). When the gingival junction level and the alveolar bone top level are not flush or substantially flush with each other, support apparatus 1 determines whether or not parameter g' (= b'/d') is smaller than 1/2, that is, whether or not a distance (d') between the gingival junction level and the root apex portion level is longer than twice as long as a distance (b') between the crown top level and the gingival junction level.

When the crown-root ratio (parameter f, g, or g') is smaller than 1/2 (YES in S9), support apparatus 1 has a portion around the measurement point shown in a first color (for example, green) for indicating that the gingiva is not recessed and one is less likely to have been affected by the periodontal disease (S10). Thereafter, support apparatus 1 makes transition to processing in S14.

When the crown-root ratio (parameter f, g, or g') is larger than 1/2 (NO in S9), on the other hand, support apparatus 1 determines whether or not the crown-root ratio (parameter f, g, or g') is smaller than 1 (S11). Specifically, when the gingival junction level and the alveolar bone top level are flush or substantially flush with each other, support apparatus 1 determines whether or not parameter f (= a/c) is smaller than 1, that is, the distance (c) between the gingival margin level and the root apex portion level is longer than the distance (a) between the crown top level and the gingival margin level. Alternatively, support apparatus 1 determines whether or not parameter g (= b/d) is smaller than 1, that is, whether or not the distance (d) between the gingival junction level (alveolar bone top level) and the root apex portion level is longer than the distance (b) between the crown top level and the gingival junction level (alveolar bone top level). When the gingival junction level and the alveolar bone top level are not flush or substantially flush with each other, support apparatus 1 determines whether or not parameter g' (=b'/d') is smaller than 1, that is, the distance (d') between the gingival junction level and the root apex portion level is longer than the distance (b') between the crown top level and the gingival junction level.

When the crown-root ratio (parameter f, g, or g') is smaller than 1 (YES in S 11), support apparatus 1 has the portion around the measurement point shown in a second color (for example, orange) to indicate that the gingiva is slightly recessed and one should pay attention not to be affected by the periodontal disease (S12). Thereafter, support apparatus 1 makes transition to processing in S14.

When the crown-root ratio (parameter f, g, or g') is larger than 1 (NO in S 11), support apparatus 1 has the portion around the measurement point shown in a third color (for example, red) to indicate that the gingiva is considerably recessed and one has most likely been affected by the periodontal disease (S13). Thereafter, support apparatus 1 makes transition to processing in S14.

In S14, support apparatus 1 determines whether or not it has shown all measurement points for at least one tooth shown in the image (S14). When support apparatus 1 has not shown all measurement points (NO in S14), it makes transition to processing in S8. When support apparatus 1 has shown all measurement points (YES in S14), on the other hand, it quits the present process.

### [Representation of Support Information]

Exemplary representation of the support information by support apparatus 1 will be described with reference to Figs. 9 to 13. Fig. 9 is a diagram for illustrating first exemplary representation of the support information by support apparatus 1.

As shown in Fig. 9, support apparatus 1 shows on display 40, a two-dimensional image of a tooth when the coronal portion is viewed from the top, with the six measurement points being added around the tooth when the coronal portion is viewed from the top. When the user moves a cursor with the use of such a peripheral device as keyboard 61 and mouse 72 to designate any measurement point, support apparatus 1 provides pop-up representation around the designated measurement point, of a parameter estimated at the designated measurement point together with a tooth number as the support information.

For example, for a tooth designated with the cursor, support apparatus 1 shows the tooth number and the depth (parameter e or e') of the periodontal pocket estimated for each of the six measurement points. As described with reference to S9 to S13 in Fig. 8, support apparatus 1 has the portion around each measurement point shown in a color based on the crown-root ratio. Furthermore, for the measurement point designated with the cursor, support apparatus 1 also has the depth (parameter e or e') of the periodontal pocket shown in a color in accordance with the crown-root ratio (f, g, or g'). In this example, for a tooth No. 36, the value (a value from 3 mm to 6 mm) of parameter e estimated for each of the six measurement points is shown. Furthermore, each measurement point is color-coded based on the crown-root ratio (f or g), and a value (6 mm) of parameter e corresponding to the measurement point designated with the cursor is shown in the color based on the crown-root ratio (f or g). In other words, support apparatus 1 provides representation in the color in accordance with an extent of recession of the gingiva indicated by the crown-root ratio (for g). The user can readily objectively know a condition of the periodontal disease.

Without being limited to the depth (parameter e or e') of the periodontal pocket, support apparatus 1 may show estimated values of various parameters for the measurement point designated with the cursor. For example, when the gingival junction level and the alveolar bone top level are flush or substantially flush with each other, support apparatus 1 may show any of parameters a to g. Alternatively, when the gingival junction level and the alveolar bone top level are not flush or substantially flush with each other, support apparatus 1 may show any of parameters b', d', e', and g'.

Though support apparatus 1 shows the value of the parameter only for a single tooth (the tooth No. 36) in the example in Fig. 9, the value of the parameter at each measurement point may be shown for a plurality of teeth or all teeth.

Support apparatus 1 can thus show on display 40, as the support information, any of parameters a to g or parameters b', d', e', and g' estimated with estimation model 50, as being superimposed on a designated position of the biological tissue. Furthermore, support apparatus 1 can show on display 40, the depth (parameter e or e') of the periodontal pocket estimated as the support information, in a color in accordance with the crown-root ratio (f, g, or g'). Such a value of each color-coded parameter shown on display 40 can be information indicating the degree of progress of the state of disease in the biological tissue in the oral cavity such as the periodontal disease.

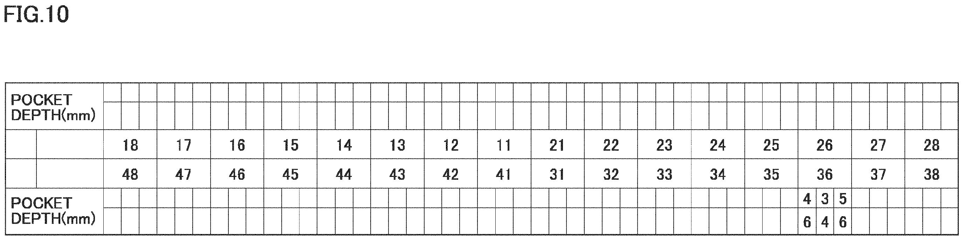

Fig. 10 is a diagram for illustrating second exemplary representation of the support information by support apparatus 1. As shown in Fig. 10, support apparatus 1 may create a chart in which depths of the periodontal pockets in the teeth are summarized and may show the created chart on display 40.

For example, for each tooth, support apparatus 1 shows as the support information, the depth (parameter e or e') of the periodontal pocket estimated for each of the six measurement points. Though support apparatus 1 shows the depth (parameter e or e') of the periodontal pocket only for a single tooth (the tooth No. 36) in the example in Fig. 10, it may show the depth (parameter e or e') of the periodontal pocket at each measurement point for a plurality of teeth or all teeth.

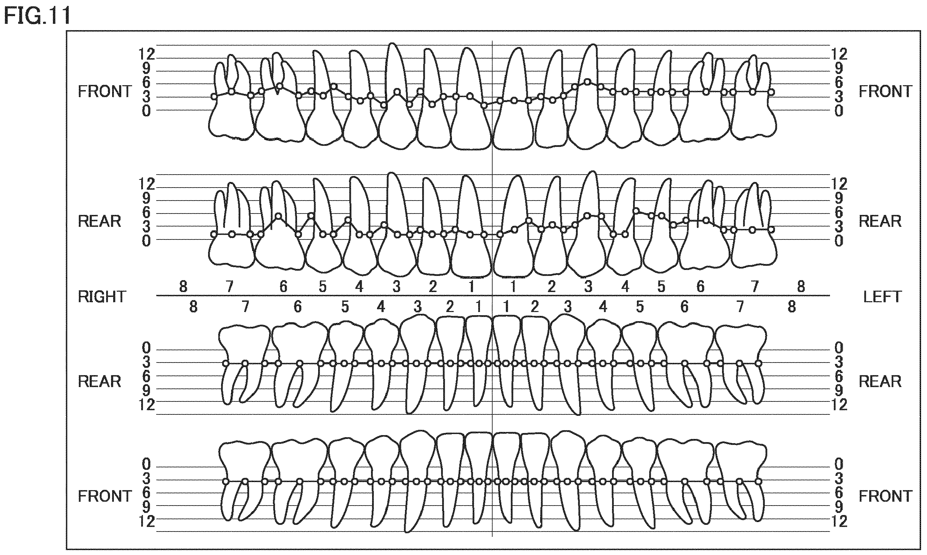

Fig. 11 is a diagram for illustrating third exemplary representation of the support information by support apparatus 1. As shown in Fig. 11, support apparatus 1 may show on display 40, a two-dimensional image simulating a dentition and show as the support information, the depth (parameter e or e') of the periodontal pocket at each measurement point in each of aligned teeth. For example, in Fig. 11, images of a front side and a rear side of each tooth included in each of an upper dentition and a lower dentition are shown on display 40, and a point is plotted at a position at the gingival junction level when the gingival margin level of each tooth is defined as "0". Though support apparatus 1 shows only the depth (parameter e or e') of the periodontal pocket of each tooth as the support information in the example in Fig. 11, it may show any of parameters a to g or any of parameters b', d', e', and g' estimated with estimation model 50.

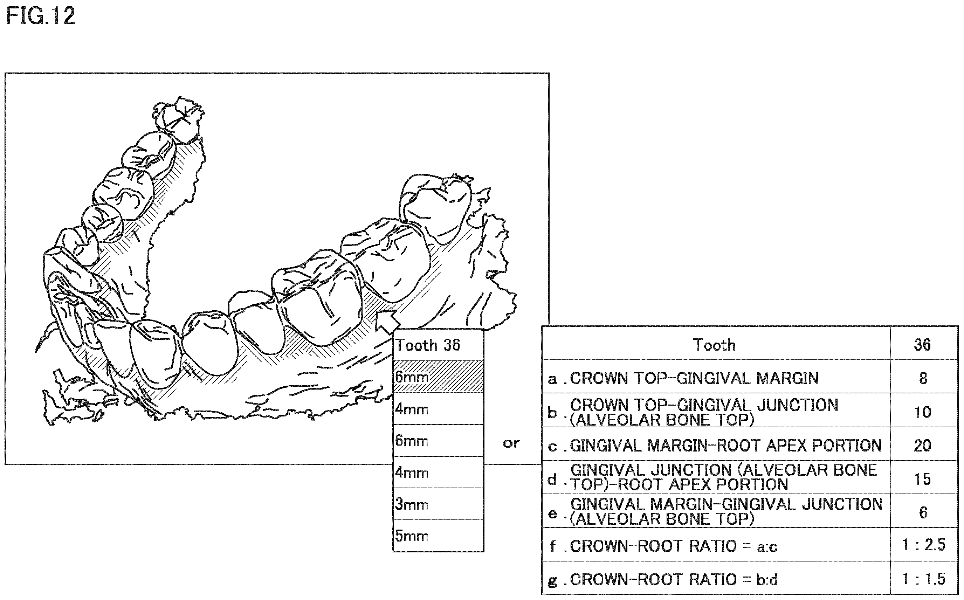

Fig. 12 is a diagram for illustrating fourth exemplary representation of the support information by the support apparatus. As shown in Fig. 12, support apparatus 1 may show on display 40, a three-dimensional image showing the dentition or the inside of the oral cavity and may provide pop-up representation around the measurement point designated by the user, of a parameter estimated at each designated measurement point, together with the tooth number as the support information.

For example, for a tooth designated with the cursor, support apparatus 1 may show the tooth number and the depth (parameter e or e') of the periodontal pocket estimated for each of the six measurement points. Alternatively, for a part of the tooth designated with the cursor, support apparatus 1 may show the depth (parameter e or e') of the periodontal pocket in a color in accordance with the crown-root ratio (f, g, or g'). In this example, for the tooth No. 36, the value (a value from 3 mm to 6 mm) of parameter e estimated for each of the six measurement points is shown. Furthermore, the value (6 mm) of parameter e corresponding to the measurement point designated with the cursor is shown in a color based on the crown-root ratio (f or g).

Support apparatus 1 may show the gingiva around each tooth as being color-coded based on the crown-root ratio (f, g, or g'). For example, support apparatus 1 may indicate to the user, magnitude of the depth (parameter e or e') of the periodontal pocket by showing the gingiva in a highlight color with the use of a heat map or the like in accordance with the crown-root ratio. The user can thus objectively know the magnitude of the depth (parameter e or e') of the periodontal pocket for each part in the dentition.

Furthermore, support apparatus 1 may calculate and show any of parameters a to g or any of parameters b', d', e', and g' for a continuous portion (more than six measurement points) around each tooth, and support apparatus 1 may calculate and show an average value or a deviation of the crown-root ratio (f, g, or g') for individual teeth. Support apparatus 1 may show the gingiva in the highlight color such that the user can objectively know the depth of the periodontal pocket.

For the measurement point designated with the cursor, support apparatus 1 may show estimated values of various parameters, without being limited to the depth (parameter e or e') of the periodontal pocket. For example, when the gingival junction level and the alveolar bone top level are flush or substantially flush with each other, support apparatus 1 may show any of parameters a to g. Alternatively, when the gingival junction level and the alveolar bone top level are not flush or substantially flush with each other, support apparatus 1 may show any of parameters b', d', e', and g'.

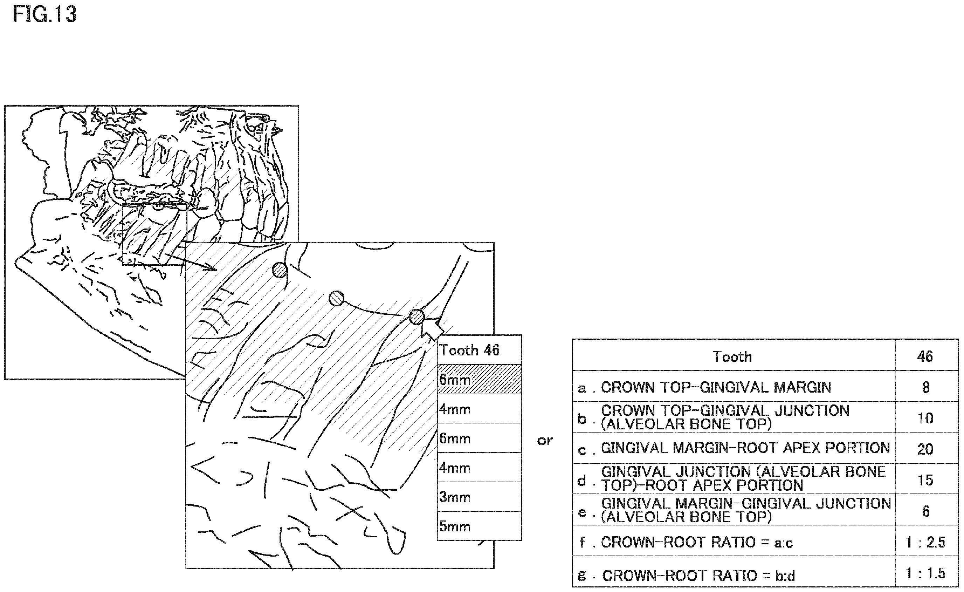

Fig. 13 is a diagram for illustrating fifth exemplary representation of the support information by the support apparatus. As shown in Fig. 13, support apparatus 1 may show on display 40, a three-dimensional combined image of the biological tissue including the tooth and the gingiva and may provide pop-up representation around the measurement point designated by the user, of the parameter estimated at the designated measurement point, together with the tooth number, as the support information.

For example, for the tooth designated with the cursor, support apparatus 1 may show the tooth number and the depth (parameter e or e') of the periodontal pocket estimated for each of the six measurement points. Alternatively, for a part of the tooth designated with the cursor, support apparatus 1 may show the depth (parameter e or e') of the periodontal pocket in a color in accordance with the crown-root ratio (f, g, or g'). In this example, for a tooth No. 46, the value (a value from 3 mm to 6 mm) of parameter e estimated for each of the six measurement points is shown. Furthermore, support apparatus 1 may show the value (6 mm) of parameter e corresponding to the measurement point designated with the cursor in a color based on the crown-root ratio (f or g).

Furthermore, support apparatus 1 may calculate and show any of parameters a to g or any of parameters b', d', e', and g' for a continuous portion (for example, more than six measurement points) around each tooth, and support apparatus 1 may calculate and show an average value or a deviation of the crown-root ratio (f, g, or g') for individual teeth. Support apparatus 1 may show the gingiva in the highlight color such that the user can objectively know the depth of the periodontal pocket.

For the measurement point designated with the cursor, support apparatus 1 may show estimated values of various parameters, without being limited to the depth (parameter e or e') of the periodontal pocket. For example, when the gingival junction level and the alveolar bone top level are flush or substantially flush with each other, support apparatus 1 may show any of parameters a to g. Alternatively, when the gingival junction level and the alveolar bone top level are not flush or substantially flush with each other, support apparatus 1 may show any of parameters b', d', e', and g'.

As set forth above, support apparatus 1 estimates, for each tooth, various parameters a to g or b', d', e', and g' based on the image data (combined image data) of the combined image generated based on the IOS data and the CT data, and presents the values of these parameters to the user. The user can thus low-invasively check the depth of the periodontal pocket without insertion of the probe in the periodontal pocket of the patient and can highly accurately check a state of progress of the periodontal disease without relying on his/her own skills.

As shown in Figs. 9 to 13, support apparatus 1 shows on display 40, a two-dimensional or three-dimensional image including a tooth, shows values of various parameters such as the depth (parameter e or e') of the periodontal pocket at the designated measurement point, or color-codes the gingiva or the value of the parameter based on the crown-root ratio (f, g, or g'). Since the user can thus readily know the degree of progress of the state of disease of the periodontal disease, the user can more easily provide explanation to the patient and more easily obtain also understanding by the patient.

### [Modification]

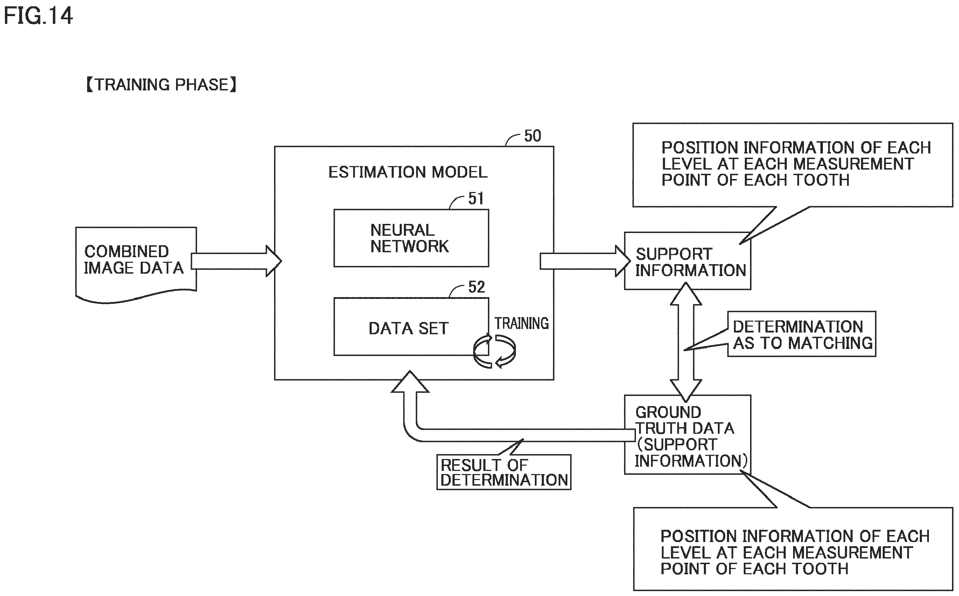

A modification of the support apparatus 1 will be described with reference to Figs. 14 to 19. In the modification, only a difference will be described, and a component identical to that described above has the same reference character allotted and description thereof will not be repeated.

A first modification will be described. Fig. 14 is a diagram for illustrating exemplary machine learning in the training phase of estimation model 50 according to the first modification. As shown in Fig. 14, in the first modification, the direction of measurement and the measurement point are not included in the training data. The position information of each level (the crown top level, the gingival margin level, the alveolar bone top level, the gingival junction level, and the root apex portion level) along the direction of measurement at each measurement point of each tooth is adopted as the support information which is the ground truth data.

When estimation model 50 receives input of the combined image data, it estimates with neural network 51, position information (support information) of each level along the direction of measurement at each measurement point of each tooth based on the combined image data. Though estimation model 50 does not receive input of the direction of measurement and the measurement point at this time, it estimates, by estimation by the estimation model itself, the position information (support information) of each level along the direction of measurement at each measurement point of each tooth. Estimation model 50 determines whether or not the estimated position information (support information) of each level along the direction of measurement at each measurement point of each tooth matches with position information (support information) of each level along the direction of measurement at each measurement point of each tooth which is the ground truth data associated with the combined image data. When they match with each other, estimation model 50 does not update data set 52, whereas when they do not match with each other, estimation model 50 updates data set 52 to optimize data set 52.

Estimation model 50 is thus trained to highly accurately estimate the position information of each level (the crown top level, the gingival margin level, the alveolar bone top level, the gingival junction level, and the root apex portion level) along the direction of measurement at each measurement point of each tooth based on the input data, by using the training data including the combined image data which is the input data and the position information (support information) of each level which is the ground truth data to optimize data set 52. In other words, estimation model 50 is trained to highly accurately estimate, without reception of input of the direction of measurement and the measurement point, the position information of each level along the direction of measurement at each measurement point of each tooth based on the inputted combined image data by machine learning also of the direction of measurement and the measurement point that it has not received.

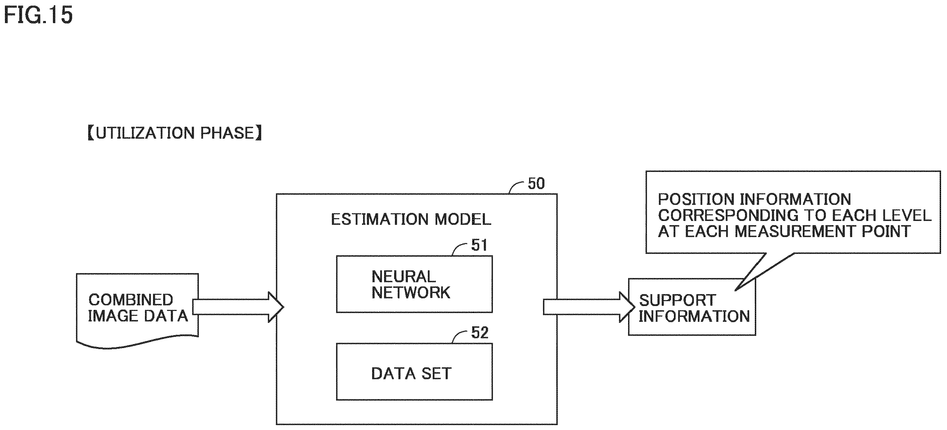

Fig. 15 is a diagram for illustrating exemplary estimation of the support information in the utilization phase of estimation model 50 according to the first modification. As shown in Fig. 15, by being trained by machine learning, when estimation model 50 according to the first modification receives input of the combined image data, estimation model 50 can estimate, as the support information, the position information of each level (the crown top level, the gingival margin level, the alveolar bone top level, the gingival junction level, and the root apex portion level) along the direction of measurement at each measurement point of each tooth based on the combined image data. Thus, according to estimation model 50 according to the first modification, without setting of the direction of measurement and the measurement point in accordance with designation by the user as in S2 and S3 in Fig. 8, the direction of measurement and the measurement point can automatically be estimated and the position information of each level along the direction of measurement at each measurement point of each tooth can highly accurately be estimated.

In the training phase, any one of the direction of measurement and the measurement point may be included in the input data, together with the combined image data. In this case, in the utilization phase, estimation model 50 can highly accurately estimate the position information of each level along the direction of measurement at each measurement point of each tooth based on the input data including any one of the direction of measurement and the measurement point and the combined image data.

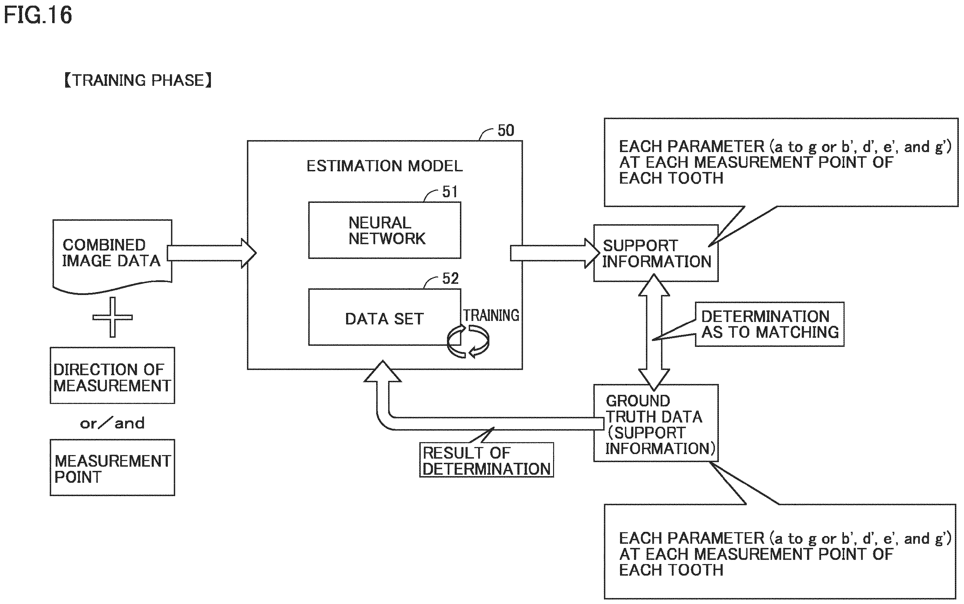

A second modification will be described. Fig. 16 is a diagram for illustrating exemplary machine learning in the training phase of estimation model 50 according to the second modification. As shown in Fig. 16, in the second modification, each parameter (a to g or b', d', e', and g') at each measurement point of each tooth is adopted as the support information. For example, when the gingival junction level and the alveolar bone top level are flush or substantially flush with each other in a tooth for which estimation is to be made, parameters a to g at each measurement point of each tooth are adopted as the support information. When the gingival junction level and the alveolar bone top level are not flush or substantially flush with each other in a tooth for which estimation is to be made, parameters b', d', e', and g' at each measurement point of each tooth are adopted as the support information.

In training of estimation model 50, machine learning is performed with the use of the training data including the combined image data, the direction of measurement, and the measurement point as the input data and the support information (a to g or b', d', e', and g') associated with the combined image data, the direction of measurement, and the measurement point as the ground truth data. Any one of the direction of measurement and the measurement point may be included in the input data, together with the combined image data.

When estimation model 50 receives input of the combined image data, the direction of measurement, and the measurement point, it estimates with neural network 51, parameters (a to g or b', d', e', and g') at each measurement point of each tooth based on the image data, the direction of measurement, and the measurement point. Estimation model 50 determines whether or not the estimated parameters (a to g or b', d', e', and g') (support information) match with the parameters (a to g or b', d', e', and g') at each measurement point of each tooth which are the ground truth data associated with the image data, the direction of measurement, and the measurement point. When they match with each other, estimation model 50 does not update data set 52, whereas when they do not match with each other, estimation model 50 updates data set 52 to optimize data set 52.

Estimation model 50 is thus trained to highly accurately estimate the parameters (a to g or b', d', e', and g') (support information) at each measurement point of each tooth based on the input data by using the training data including the combined image data, the direction of measurement, and the measurement point which are the input data and the parameters (a to g or b', d', e', and g') (support information) at each measurement point of each tooth which is the ground truth data to optimize data set 52.

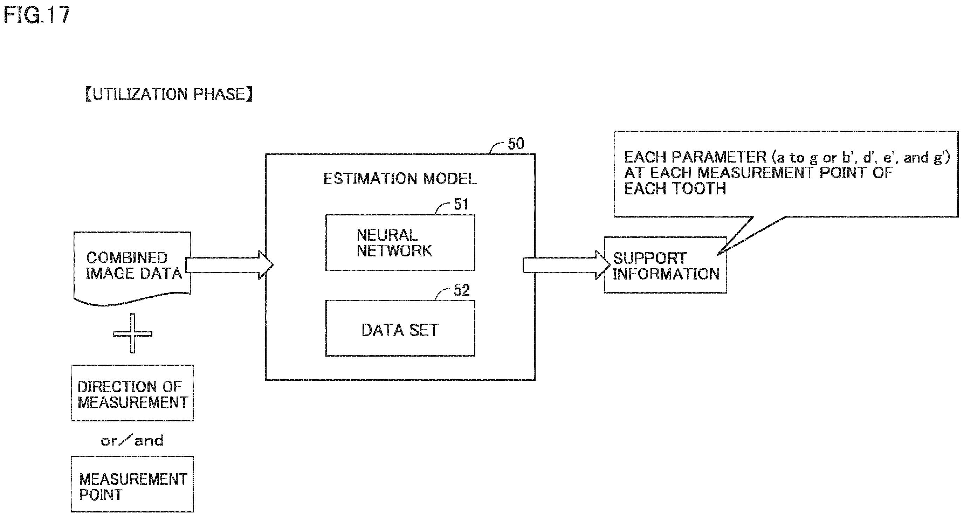

Fig. 17 is a diagram for illustrating exemplary estimation of the support information in the utilization phase of estimation model 50 according to the second modification. As shown in Fig. 17, by being trained by machine learning, when estimation model 50 according to the second modification receives input of the image data, the direction of measurement, and the measurement point, estimation model 50 can highly accurately estimate, as the support information, the parameters (a to g or b', d', e', and g') at each measurement point of each tooth based on the combined image data, the direction of measurement, and the measurement point.

In the training phase, at least one of the plurality of parameters (a to g or b', d', e', and g') may be included in the support information which is the ground truth data. In this case, in the utilization phase, estimation model 50 estimates as the support information, at least one of the plurality of parameters (a to g or b', d', e', and g') at each measurement point of each tooth based on the input data including the combined image data, the direction of measurement, and the measurement point.

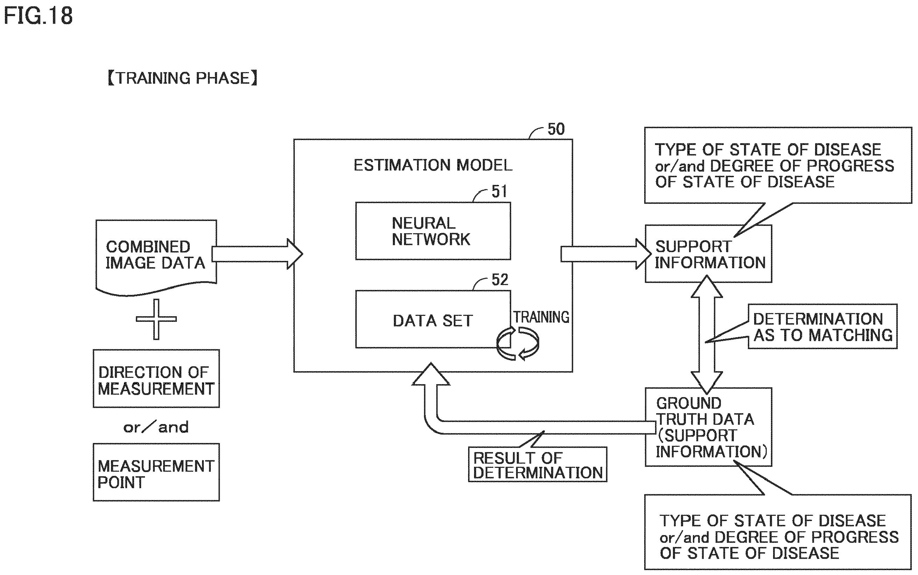

A third modification will be described. Fig. 18 is a diagram for illustrating exemplary machine learning in the training phase of estimation model 50 according to the third modification. As shown in Fig. 18, in the third modification, a type of the state of disease and a degree of progress of the state of disease in the biological tissue in the oral cavity are adopted as the support information.

In training of estimation model 50, machine learning is performed with the use of the training data including the combined image data, the direction of measurement, and the measurement point as the input data and the support information (the type of the state of disease and the degree of progress of the state of disease) associated with the image data, the direction of measurement, and the measurement point as the ground truth data. Any one of the direction of measurement and the measurement point may be included in the input data, together with the image data.

When estimation model 50 receives input of the combined image data, the direction of measurement, and the measurement point, it estimates with neural network 51, the type of the state of disease and the degree of progress of the state of disease in the biological tissue in the oral cavity based on the image data, the direction of measurement, and the measurement point. Estimation model 50 determines whether or not the estimated type of the state of disease and the degree of progress of the state of disease (support information) match with the type of the state of disease and the degree of progress of the state of disease in the biological tissue in the oral cavity which is the ground truth data associated with the image data, the direction of measurement, and the measurement point. When they match with each other, estimation model 50 does not update data set 52, whereas when they do not match with each other, estimation model 50 updates data set 52 to optimize data set 52.

Estimation model 50 is thus trained to highly accurately estimate the type of the state of disease and the degree of progress of the state of disease in the biological tissue in the oral cavity based on the input data by using the training data including the combined image data, the direction of measurement, and the measurement point which are the input data and the type of the state of disease and the degree of progress of the state of disease (support information) in the biological tissue in the oral cavity which are the ground truth data to optimize data set 52.

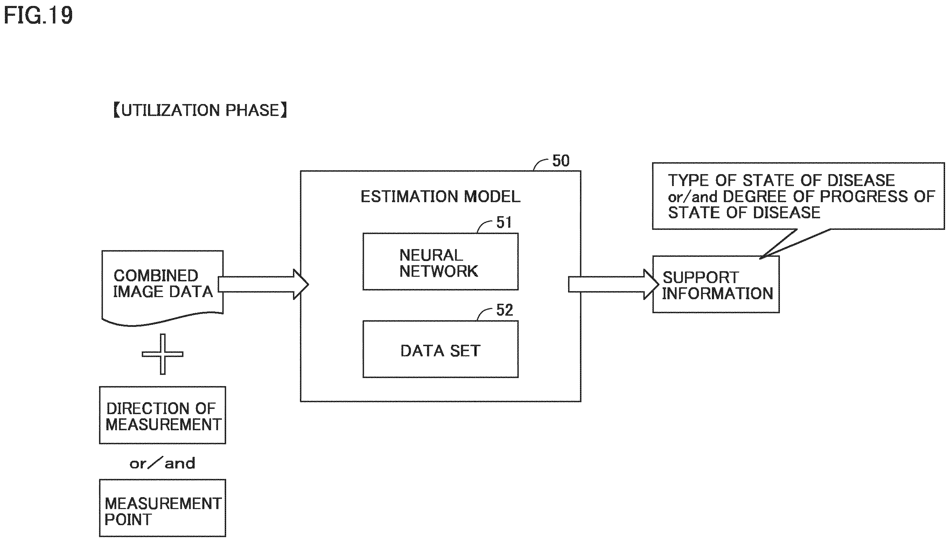

Fig. 19 is a diagram for illustrating exemplary estimation of the support information in the utilization phase of estimation model 50 according to the third modification. As shown in Fig. 19, by being trained by machine learning, when estimation model 50 according to the third modification receives input of the combined image data, the direction of measurement, and the measurement point, estimation model 50 can highly accurately estimate, as the support information, the type of the state of disease and the degree of progress of the state of disease in the biological tissue in the oral cavity based on the image data, the direction of measurement, and the measurement point.

In the training phase, at least one of the type of the state of disease and the degree of progress of the state of disease may be included in the support information which is the ground truth data. In this case, in the utilization phase, estimation model 50 estimates at least one of the type of the state of disease and the degree of progress of the state of disease as the support information, based on the input data including the combined image data, the direction of measurement, and the measurement point.

As set forth above, support apparatus 1 may use estimation model 50 to estimate, as the support information, at least one of the plurality of levels (the crown top level, the gingival margin level, the alveolar bone top level, the gingival junction level, and the root apex portion level), estimate at least one of the parameters (a to g or b', d', e', and g') without estimation of the level, or estimate at least one of the type of the state of disease and the degree of progress of the state of disease of the periodontal disease without estimation of the level and the parameter.

In the tooth affected by the periodontal disease, the gingiva lowers, and consequently, the surface portion of the tooth is exposed more than in the tooth not affected by the periodontal disease. In other words, depending on whether or not the tooth is affected by the periodontal disease, the color of the surface of the biological tissue including the tooth and the gingiva is different. Support apparatus 1 then may adopt as the input data for the training data, together with the combined image data, color information indicating the color of the surface of the biological tissue included in the three-dimensional data obtained by the three-dimensional scanner to perform machine learning. In this case, support apparatus 1 can highly accurately estimate the degree of progress of the state of disease of the periodontal disease based on the color of the surface of the biological tissue in addition to the combined image data.

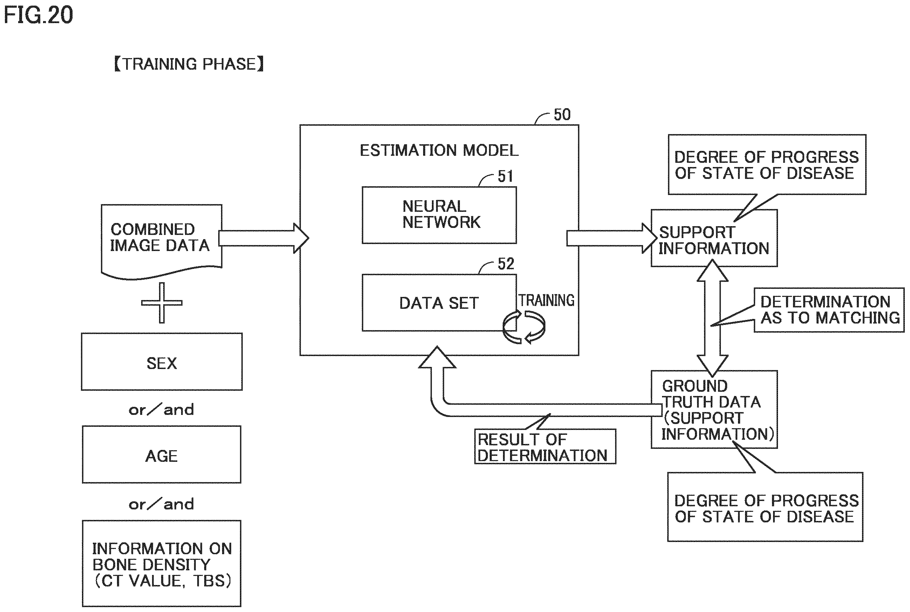

An alternative or additional configuration of the support apparatus 1 will be described with reference to Figs. 20 to 22. Only a difference of support apparatus 1 from the support apparatus 1 described above will be described, and a component identical to that of the support apparatus 1 described above has the same reference character allotted and description thereof will not be repeated.

Fig. 20 is a diagram for illustrating exemplary machine learning in the training phase of estimation model 50. As shown in Fig. 20, at least one of the sex, the age, and information on a bone density of a patient having the biological tissue shown based on combined image data is included in the training data, in addition to the combined image data. The information on the bone density includes at least one of a CT value (a monochrome image density value in the CT image) of the jaw bone around an individual tooth and a trabecular bone score (TBS) of the jaw bone around the individual tooth. In addition, the degree of progress of the state of disease in the biological tissue in the oral cavity is adopted as the support information.

Depending on the sex, the age, and the information (the CT value and the trabecular bone score) on the bone density of the patient, the degree of progress of the state of disease of the periodontal disease tends to be different. For example, it has been said that women are more prone to the periodontal disease than men. Aging also makes people vulnerable to the periodontal disease. Furthermore, the lower bone density makes people more vulnerable to the periodontal disease. Therefore, by adoption as the training data, of at least one of the sex, the age, and the information (the CT value and the trabecular bone score) on the bone density of the patient for the input data to estimation model 50, estimation model 50 can estimate the degree of progress of the state of disease of the periodontal disease.

For example, in training of estimation model 50, machine learning is performed with the training data including the combined image data and the sex, the age, and the information (the CT value and the trabecular bone score) on the bone density as the input data and the support information (the degree of progress of the state of disease) associated with the image data and the sex, the age, and the information on the bone density as the ground truth data. Any one of the direction of measurement and the measurement point may be included in the input data.

When estimation model 50 receives input of the combined image data and the sex, the age, and the information (the CT value and the trabecular bone score) on the bone density, it estimates with neural network 51, the degree of progress of the state of disease in the biological tissue in the oral cavity based on the image data and the sex, the age, and the information on the bone density. Estimation model 50 determines whether or not the estimated degree of progress of the state of disease (support information) matches with the degree of progress of the state of disease in the biological tissue in the oral cavity which is the ground truth data associated with the image data and the sex, the age, and the information on the bone density. When they match with each other, estimation model 50 does not update data set 52, whereas when they do not match with each other, estimation model 50 updates data set 52 to optimize data set 52.

Estimation model 50 is thus trained to highly accurately estimate the degree of progress of the state of disease in the biological tissue in the oral cavity based on the input data, by using the training data including the combined image data and the sex, the age, and the information (the CT value and the trabecular bone score) on the bone density which are the input data and the degree of progress of the state of disease in the biological tissue in the oral cavity (support information) which is the ground truth data to optimize data set 52.

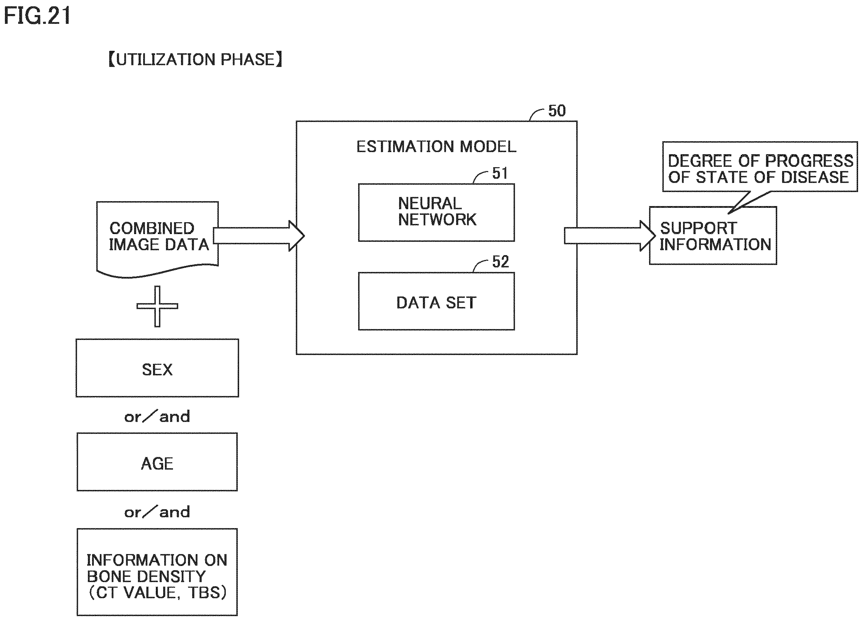

Fig. 21 is a diagram for illustrating exemplary estimation of the support information in the utilization phase of estimation model 50. As shown in Fig. 21, by being trained by machine learning, when estimation model 50 receives input of the combined image data and the sex, the age, and the information on the bone density, estimation model 50 can highly accurately estimate the degree of progress of the state of disease in the biological tissue in the oral cavity based on the image data and the sex, the age, and the information (the CT value and the trabecular bone score) on the bone density.

In the training phase, at least one of the sex, the age, and the information (the CT value and the trabecular bone score) on the bone density may be included in the input data in addition to the combined image data. In this case, in the utilization phase, estimation model 50 estimates the degree of progress of the state of disease as the support information based on the combined image data and at least one of the sex, the age, and the information (the CT value and the trabecular bone score) on the bone density.

Thus, by adopting as the input data for the training data, at least one of the sex, the age, and the information (the CT value and the trabecular bone score) on the bone density of the patient that may affect the degree of progress of the state of disease of the periodontal disease to perform machine learning, support apparatus 1 can highly accurately estimate the degree of progress of the state of disease of the periodontal disease without relying on experiences of the operator while burdens imposed on the operator are mitigated. Furthermore, by estimation and recording of the degree of progress of the state of disease of the periodontal disease of the patient with the use of support apparatus 1 every year in a universal dental checkup which will be introduced in the future, the user can also predict the degree of progress of the state of disease in the future based on records.

Furthermore, estimation model 50 may be trained by machine learning with the training data including yearly records of the degree of progress of the state of disease as described above as the input data and the degree of progress of the state of disease as the ground truth data. In this case, when estimation model 50 receives input of the current degree of progress of the state of disease, it can estimate also the degree of progress of the state of disease in the future based on the current degree of progress of the state of disease.

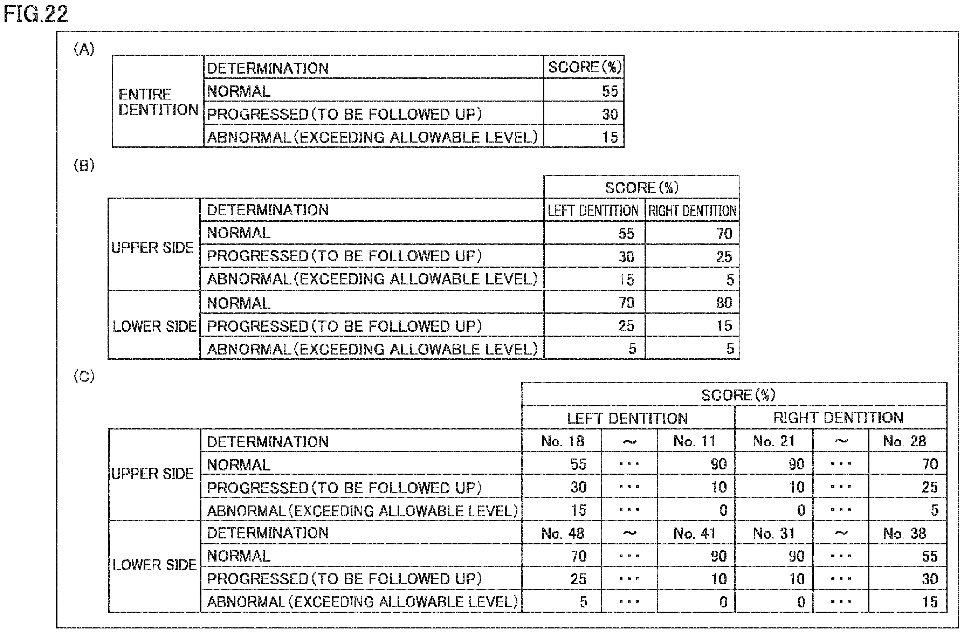

Fig. 22 is a diagram for illustrating exemplary output of the support information by support apparatus 1. As shown in Fig. 22, support apparatus 1 may convert the degree of progress of the state of disease of the periodontal disease estimated with the use of estimation model 50 into a score based on a prescribed criterion, and may show the calculated score on display 40.

For example, as shown in Fig. 22 (A), support apparatus 1 may estimate the degree of progress of the state of disease of the periodontal disease for the entire dentition, and calculate and show a score corresponding to the degree of progress of the state of disease based on the prescribed criterion for each of "normal", "progressed (to be followed up)," and "abnormal (exceeding allowable level)." As shown in Fig. 22 (B), support apparatus 1 may estimate the degree of progress of the state of disease of the periodontal disease for each of an upper left dentition, an upper right dentition, a lower left dentition, and a lower right dentition, and calculate and show a score corresponding to the degree of progress of the state of disease based on the prescribed criterion for each of "normal", "progressed (to be followed up)," and "abnormal (exceeding allowable level)." As shown in Fig. 22 (C), support apparatus 1 may estimate the degree of progress of the state of disease of the periodontal disease for each tooth included in each of the upper left dentition, the upper right dentition, the lower left dentition, and the lower right dentition, and calculate and show a score corresponding to the degree of progress of the state of disease based on the prescribed criterion for each of "normal", "progressed (to be followed up)," and "abnormal (exceeding allowable level)."

Support apparatus 1 may show the score corresponding to the degree of progress of the state of disease in an image of the tooth as shown in Figs. 9 to 13. For example, support apparatus 1 may show the gingiva in the highlight color, with the use of a heat map in accordance with the degree of progress of the state of disease in the image of the biological tissue including the tooth and the gingiva as shown in Fig. 12 or 13. Support apparatus 1 may show to the user, simulation information that shows prediction of future change of the degree of progress of the state of disease (support information) by presenting such an image showing the degree of progress of the state of disease in a time series manner every elapsed time in the future (for example, one year later, two years later, or the like).

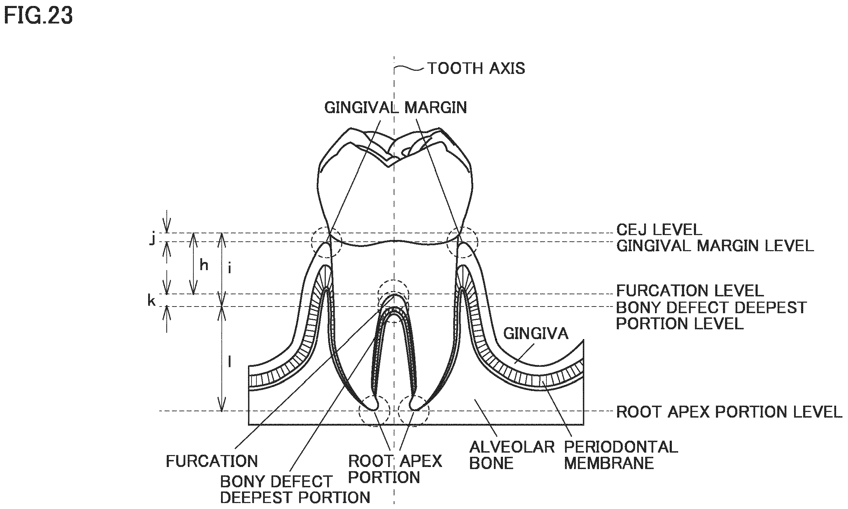

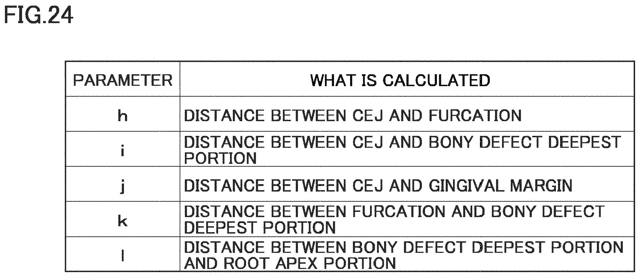

An alternative or additional configuration of the support apparatus 1 will be described with reference to Figs. 23 and 24. Only a difference of the support apparatus 1 from the support apparatus 1 described above will be described, and a component identical to that of the support apparatus 1 described above has the same reference character allotted and description thereof will not be repeated.

Figs. 23 and 24 are each a diagram for illustrating a parameter estimated in support apparatus 1. Fig. 23 shows a vertical cross-section of the biological tissue including the tooth and the gingiva.

As shown in Fig. 23, support apparatus 1 estimates with estimation model 50, at each measurement point of each tooth, position information (X, Y, Z) corresponding to each of a CEJ level, the gingival margin level, a furcation level, a bony defect deepest portion level, and the root apex portion level as a plurality of levels indicating positions of the biological tissues present along the direction of measurement. The CEJ level refers to a level corresponding to a position (for example, a height) of a junction (cement enamel junction) between cementum located between a dental root and a periodontal membrane and enamel that covers the tooth exposed outside the gingiva. The furcation level refers to a level corresponding to a position (for example, a height) of the furcation. The bony defect deepest portion level refers to a level corresponding to a position (for example, a height) of a deepest defect portion of the alveolar bone when the furcation develops a lesion and there is a space between the furcation and the alveolar bone.

Support apparatus 1 can derive the degree of progress of the state of disease at the furcation that has developed the lesion by estimating the CEJ level, the gingival margin level, the furcation level, the bony defect deepest portion level, and the root apex portion level at each measurement point of each tooth. Specifically, as shown in Figs. 23 and 24, support apparatus 1 calculates each of parameters h to 1. Parameter h is a value indicating a distance along the direction of measurement between the CEJ level and the furcation level. Parameter i is a value indicating a distance along the direction of measurement between the CEJ level and the bony defect deepest portion level. Parameter j refers to a value indicating a distance along the direction of measurement between the CEJ level and the gingival margin level. Parameter k is a value indicating a distance along the direction of measurement between the furcation level and the bony defect deepest portion level. Parameter 1 is a value indicating a distance along the direction of measurement between the bony defect deepest portion level and the root apex portion level.

The user can diagnose the state of disease at the furcation that has developed the lesion for each measurement point of each tooth with the use of various parameters estimated by support apparatus 1. For example, the user can diagnose the state of disease at the furcation that has developed the lesion, in accordance with known Glickman's furcation classification, Lindhe's furcation classification, or Tarnow & Fletcher's classification, based on at least one of parameters h to 1 estimated by support apparatus 1.

The user can thus low-invasively diagnose the state of disease involved with the lesion at the furcation in the biological tissue including the tooth and the gingiva in a short period of time by knowing various parameters estimated by support apparatus 1.

Support apparatus 1 may use estimation model 50 to estimate at least one of the plurality of levels (the CEJ level, the gingival margin level, the furcation level, the bony defect deepest portion level, and the root apex portion level) as the diagnosis information, estimate at least one of parameters h to 1 without estimating the level, or estimate the degree of progress of the state of disease at the furcation that has developed the lesion without estimating the level and the parameter.

Support apparatus 1 described above may include a feature and a function thereof, alone or in combination. Furthermore, support apparatus 1 may include a feature and a function in the modifications described above, alone or in combination.

It should be understood that the above description is illustrative and non-restrictive in every respect. The present disclosure is only defined by the claimed subject-matter. It is intended to include any modifications within the claimed subject-matter. The configurations described above can be combined as appropriate.

## Claims

1. A support apparatus (1) that is configured to support diagnosis of a state of disease in a biological tissue in an oral cavity including at least a tooth and a gingiva, the support apparatus (1) comprising:
an input unit (14) that is configured to receive input of image data showing a three-dimensional geometry of the biological tissue; and
a computing unit (11) that is configured to derive support information including information on positions of at least the tooth and the gingiva relative to each other, by using the image data inputted from the input unit (14) and an estimation model (50) for support of diagnosis of the state of disease in the biological tissue based on the image data, the estimation model (50) being trained by machine learning to derive the support information.

2. The support apparatus (1) according to claim 1, wherein
the support information includes position information corresponding to each of a plurality of levels each indicating a position of the biological tissue present along a prescribed direction of measurement at a prescribed measurement point in the biological tissue.

3. The support apparatus (1) according to claim 1 or 2, wherein
the support information includes a distance between a plurality of levels each indicating a position of the biological tissue present along a prescribed direction of measurement at a prescribed measurement point in the biological tissue.

4. The support apparatus (1) according to any one of claims 1 to 3, wherein
the computing unit (11) is configured to
derive as the support information, position information corresponding to each of a plurality of levels each indicating a position of the biological tissue present along a prescribed direction of measurement at a prescribed measurement point in the biological tissue,
calculate a distance between the plurality of levels based on the position information corresponding to each of the plurality of levels, and
calculate at least one of information indicating a type of the state of disease and information indicating a degree of progress of the state of disease based on the distance between the plurality of levels.

5. The support apparatus (1) according to any one of claims 1 to 4, wherein
the support information includes at least one of information indicating a type of the state of disease and information indicating a degree of progress of the state of disease.

6. The support apparatus (1) according to any one of claims 1 to 5, wherein
the estimation model is trained by the machine learning with training data including the image data and the support information associated with the image data.

7. The support apparatus (1) according to any one of claims 2 to 6, wherein
the prescribed measurement point is a point located around the tooth when a coronal portion included in the tooth is viewed from top.

8. The support apparatus (1) according to any one of claims 2 to 7, wherein
the prescribed direction of measurement is a direction along a tooth axis of the tooth.

9. The support apparatus (1) according to any one of claims 2 to 8, wherein
the plurality of levels indicate any position in the biological tissue, of a top of a coronal portion, a margin of the gingiva, a top of an alveolar bone, a junction between the tooth and the gingiva, a root apex portion, a furcation, a junction between cementum and enamel of the tooth, and a deepest portion of a defect portion of the alveolar bone that is lost in the furcation.

10. The support apparatus (1) according to any one of claims 2 to 9, wherein
the computing unit (11) is configured to derive with the estimation model (50), the support information for a site along the prescribed direction of measurement at the prescribed measurement point in the biological tissue based on the prescribed measurement point and the prescribed direction of measurement in addition to the image data inputted from the input unit.

11. The support apparatus (1) according to any one of claims 1 to 10, wherein
the computing unit (11) is configured to derive with the estimation model (50), information indicating a degree of progress of the state of disease as the support information, based on at least one of a sex, an age, and information on a bone density of a patient having the biological tissue, in addition to the image data inputted from the input unit.

12. The support apparatus (1) according to any one of claims 1 to 11, wherein
the image data is generated based on optical scanner data obtained by scanning by an optical scanner, the optical scanner data including position information of each point in a point group indicating a surface of the biological tissue, and computed tomography, CT, data obtained by CT scanning of the biological tissue.

13. The support apparatus (1) according to claim 12, wherein
the optical scanner data includes information indicating a color of the surface of the biological tissue.

14. The support apparatus (1) according to any one of claims 1 to 13, wherein
the computing unit (11) is configured to cause a display (40) to show the support information as being superimposed on a designated position in the biological tissue.

15. The support apparatus (1) according to claim 14, wherein
the computing unit (11) is configured to cause the display (40) to show the support information in a color in accordance with the support information.

16. The support apparatus (1) according to claim 14 or 15, wherein
the computing unit (11) is configured to cause the display (40) to show, based on the support information, simulation information that shows prediction of future change of the support information.

17. A support method of supporting, by a computer (11), diagnosis of a state of disease in a biological tissue in an oral cavity including at least a tooth and a gingiva, the support method comprising, as processing to be performed by the computer:
obtaining image data showing a three-dimensional geometry of the biological tissue; and
deriving support information including information on positions of at least the tooth and the gingiva relative to each other, by using the image data obtained in the obtaining of image data and an estimation model (50) for support of diagnosis of the state of disease in the biological tissue based on the image data, the estimation model being trained by machine learning to derive the support information.

18. A support program (30) for support, by a computer (11), of diagnosis of a state of disease in a biological tissue in an oral cavity including at least a tooth and a gingiva, the support program causing the computer to perform:
obtaining image data showing a three-dimensional geometry of the biological tissue; and
deriving support information including information on positions of at least the tooth and the gingiva relative to each other, by using the image data obtained in the obtaining of image data and an estimation model (50) for support of diagnosis of the state of disease in the biological tissue based on the image data, the estimation model being trained by machine learning to derive the support information.
